# EUROPEAN PATENT APPLICATION

(11) **EP 0 995 759 A1**
(43) Date of publication of application: **26.04.2000**
(21) Application number: 99305510.2
(22) Date of filing: 12.07.1999
(51) Int. Cl.: C07K 14/515, C07K 14/47, C12N 15/12, A61K 38/18, G01N 33/53

(54) **Angiopoietin related gene sequence scarface 2**

(30) Priority: 24.07.1998 US 94033 P
(71) Applicant: ELI LILLY AND COMPANY, Indianapolis, Indiana 46285 (US)
(72) Inventor: Leonard, Rebecca Ann, indianapolis, Indiana 46260 (US); Rosteck, Paul Robert, Jr., Indianapolis, Indiana 46237 (US); Sankhavaram, Patanjali Raghavacharya, Carmel, Indiana 46033 (US); Burgett, Stanley Gene, Indianapolis, Indiana 46227 (US)
(74) Representative: Denholm, Anna Marie

(57) **Abstract**

The invention provides isolated Scarface 2 nucleic acid compounds, proteins and fragments thereof, said Scarface 2 proteins being related to the angiopoietin family. Also provided are vectors and transformed heterologous host cells for expressing Scarface 2, methods for identifying compounds that bind and/or modulate the activity of said Scarface 2 proteins, as well as methods for treating cancer, and pharmaceutical compositions.

### Annex to the description

SEQUENCE LISTING

<110> Burgett, Stanley G.
      Leonard, Rebecca A.
      Rosteck Jr., Paul R.
      Sankhavaram, Patanjali R.

<120> Angiopoietin Related Gene Sequence Scarface 2

<130> X-12292

<140>
<141>

<160> 3

<170> PatentIn Ver. 2.0

<210> 1
<211> 2393
<212> DNA
<213> Homo sapiens

<220>
<221> CDS
<222> (357)..(1829)

<400> 1
atacaatctg ggtcagctgc agctggttac tgcatttctc catgtggcag acagagcaaa 60

gccacaacgc tttctctgct ggattaaaga cggcccacag accagaactt ccactatact 120

acttaaaatt acataggtgg cttgtcaaat tcaattgatt agtattgtaa aaggaaaaag 180

aagttccttc ttacagcttg gattcaacgg tccaaaacaa aaatgcagct gccattaaag 240

tcacagatga acaaacttct acactgattt ttaaaatcaa gaataagggc agcaagtttc 300

tggattcact gaatcaacag acacaaaaag acatcatttt acaacctcat ttcaaa atg 359
Met
1

aag act ttt acc tgg acc cta ggt gtg cta ttc ttc cta cta gtg gac 407
Lys Thr Phe Thr Trp Thr Leu Gly Val Leu Phe Phe Leu Leu Val Asp
            5                   10                  15

act gga cat tgc aga ggt gga caa ttc aaa att aaa aaa ata aac cag 455
Thr Gly His Cys Arg Gly Gly Gln Phe Lys Ile Lys Lys Ile Asn Gln
        20                  25                  30

aga aga tac cct cgt gcc aca gat ggt aaa gag gaa gca aag aaa tgt 503
Arg Arg Tyr Pro Arg Ala Thr Asp Gly Lys Glu Glu Ala Lys Lys Cys
    35                  40                  45

gca tac aca ttc ctg gta cct gaa caa aga ata aca ggg cca atc tgt 551
Ala Tyr Thr Phe Leu Val Pro Glu Gln Arg Ile Thr Gly Pro Ile Cys
50                  55                  60                  65

gtc aac acc aag ggg caa gat gca agt acc att aaa gac atg atc acc 599
Val Asn Thr Lys Gly Gln Asp Ala Ser Thr Ile Lys Asp Met Ile Thr
                70                  75                  80

agg atg gac ctt gaa aac ctg aag gat gtg ctc tcc agg cag aag cgg 647
Arg Met Asp Leu Glu Asn Leu Lys Asp Val Leu Ser Arg Gln Lys Arg
            85                  90                  95

gag ata gat gtt ctg caa ctg gtg gtg gat gta gat gga aac att gtg   695
Glu Ile Asp Val Leu Gln Leu Val Val Asp Val Asp Gly Asn Ile Val
        100                 105                 110

aat gag gta aag ctg ctg aga aag gaa agc cgt aac atg aac tct cgt   743
Asn Glu Val Lys Leu Leu Arg Lys Glu Ser Arg Asn Met Asn Ser Arg
    115                 120                 125

gtt act caa ctc tat atg caa tta tta cat gag att atc cgt aag agg   791
Val Thr Gln Leu Tyr Met Gln Leu Leu His Glu Ile Ile Arg Lys Arg
130                 135                 140                 145

gat aat tca ctt gaa ctt tcc caa ctg gaa aac aaa atc ctc aat gtc   839
Asp Asn Ser Leu Glu Leu Ser Gln Leu Glu Asn Lys Ile Leu Asn Val
                150                 155                 160

acc aca gaa atg ttg aag atg gca aca aga tac agg gaa cta gag gtg   887
Thr Thr Glu Met Leu Lys Met Ala Thr Arg Tyr Arg Glu Leu Glu Val
            165                 170                 175

aaa tac gct tcc ttg act gat ctt gtc aat aac caa tct gtg atg atc   935
Lys Tyr Ala Ser Leu Thr Asp Leu Val Asn Asn Gln Ser Val Met Ile
        180                 185                 190

act ttg ttg gaa gaa cag tgc ttg agg ata ttt tcc cga caa gac acc   983
Thr Leu Leu Glu Glu Gln Cys Leu Arg Ile Phe Ser Arg Gln Asp Thr
    195                 200                 205

cat gtg tct ccc cca ctt gtc cag gtg gtg cca caa cat att cct aac   1031
His Val Ser Pro Pro Leu Val Gln Val Val Pro Gln His Ile Pro Asn
210                 215                 220                 225

agc caa cag tat act cct ggt ctg ctg gga ggt aac gag att cag agg   1079

Ser Gln Gln Tyr Thr Pro Gly Leu Leu Gly Gly Asn Glu Ile Gln Arg
                230                 235                 240

gat cca ggt tat ccc aga gat tta atg cca cca cct gat ctg gca act 1127
Asp Pro Gly Tyr Pro Arg Asp Leu Met Pro Pro Pro Asp Leu Ala Thr
            245                 250                 255

tct ccc acc aaa agc cct ttc aag ata cca ccg gta act ttc atc aat 1175
Ser Pro Thr Lys Ser Pro Phe Lys Ile Pro Pro Val Thr Phe Ile Asn
        260                 265                 270

gaa gga cca ttc aaa gac tgt cag caa gca aaa gaa gct ggg cat tcg 1223
Glu Gly Pro Phe Lys Asp Cys Gln Gln Ala Lys Glu Ala Gly His Ser
    275                 280                 285

gtc agt ggg att tat atg att aaa cct gaa aac agc aat gga cca atg 1271
Val Ser Gly Ile Tyr Met Ile Lys Pro Glu Asn Ser Asn Gly Pro Met
290                 295                 300                 305

cag tta tgg tgt gaa aac agt ttg gac cct ggg ggt tgg act gtt att 1319
Gln Leu Trp Cys Glu Asn Ser Leu Asp Pro Gly Gly Trp Thr Val Ile
                310                 315                 320

cag aaa aga aca gac ggc tct gtc aac ttc ttc aga aat tgg gaa aat 1367
Gln Lys Arg Thr Asp Gly Ser Val Asn Phe Phe Arg Asn Trp Glu Asn
            325                 330                 335

tat aag aaa ggg ttt gga aac att gac gga gaa tac tgg ctt gga ctg 1415
Tyr Lys Lys Gly Phe Gly Asn Ile Asp Gly Glu Tyr Trp Leu Gly Leu
        340                 345                 350

gaa aat atc tat atg ctt agc aat caa gat aat tac aag tta ttg att 1463
Glu Asn Ile Tyr Met Leu Ser Asn Gln Asp Asn Tyr Lys Leu Leu Ile

355                 360                 365

gaa tta gaa gac tgg agt gat aaa aaa gtc tat gca gaa tac agc agc    1511
Glu Leu Glu Asp Trp Ser Asp Lys Lys Val Tyr Ala Glu Tyr Ser Ser
370                 375                 380                 385

ttt cgt ctg gaa cct gaa agt gaa ttc tat aga ctg cgc ctg gga act    1559
Phe Arg Leu Glu Pro Glu Ser Glu Phe Tyr Arg Leu Arg Leu Gly Thr
                390                 395                 400

tac cag gga aat gca ggg gat tct atg atg tgg cat aat ggt aaa caa    1607
Tyr Gln Gly Asn Ala Gly Asp Ser Met Met Trp His Asn Gly Lys Gln
            405                 410                 415

ttc acc aca ctg gac aga gat aaa gat atg tat gca gga aac tgc gcc    1655
Phe Thr Thr Leu Asp Arg Asp Lys Asp Met Tyr Ala Gly Asn Cys Ala
        420                 425                 430

cac ttt cat aaa gga ggc tgg tgg tac aat gcc tgt gca cat tct aac    1703
His Phe His Lys Gly Gly Trp Trp Tyr Asn Ala Cys Ala His Ser Asn
    435                 440                 445

cta aat gga gta tgg tac aga gga ggc cat tac aga agc aag cac caa    1751
Leu Asn Gly Val Trp Tyr Arg Gly Gly His Tyr Arg Ser Lys His Gln
450                 455                 460                 465

gat gga att ttc tgg gcc gaa tac aga ggc ggg tca tac tcc tta aga    1799
Asp Gly Ile Phe Trp Ala Glu Tyr Arg Gly Gly Ser Tyr Ser Leu Arg
                470                 475                 480

gca gtt cag atg atg atc aag cct att gac tgaagagaga cactcgccaa      1849
Ala Val Gln Met Met Ile Lys Pro Ile Asp
            485                 490

tttaaatgac acagaacttt gtacttttca gctcttaaaa atgtaaatgt tacatgtata 1909

ttacttggca caatttattt ctacacataa agtttttaaa atgaatttta ccgtaactat 1969

aaaagggaac ctataaatgt agtttcatct gtcgtcaatt actgcagaaa attatgtgta 2029

tccacaacct agttatttta aaaattatgt tgactaaata caaagtttgt tttctaaaat 2089

gtaaatattt gccacaatgt aaagcaaatc ttagctatat tttaaatcat aaataacatg 2149

ttcaagatac ttaacaattt atttaaaatc taagattgct ctaacgtcta gtgaaaaaaa 2209

tatttttaaa atttcagcca aataatgcat tttatttata aaaatacaga cagaaaatta 2269

gggagaaacc tctagttttg ccaatagaaa atgcttcttc cattgaataa aagttatttc 2329

aaattgaaaa aaaaaaaaaa aaaaaaaaaa aaaaaaaaaa aaaaaaaaaa aaaaaaaaaa 2389

aaaa 2393

<210> 2

<211> 491

<212> PRT

<213> Homo sapiens

<400> 2

Met Lys Thr Phe Thr Trp Thr Leu Gly Val Leu Phe Phe Leu Leu Val
1               5                   10                  15

Asp Thr Gly His Cys Arg Gly Gly Gln Phe Lys Ile Lys Lys Ile Asn
            20                  25                  30

Gln Arg Arg Tyr Pro Arg Ala Thr Asp Gly Lys Glu Glu Ala Lys Lys
        35                  40                  45
Cys Ala Tyr Thr Phe Leu Val Pro Glu Gln Arg Ile Thr Gly Pro Ile
    50                  55                  60
Cys Val Asn Thr Lys Gly Gln Asp Ala Ser Thr Ile Lys Asp Met Ile
65                  70                  75                  80
Thr Arg Met Asp Leu Glu Asn Leu Lys Asp Val Leu Ser Arg Gln Lys
                85                  90                  95
Arg Glu Ile Asp Val Leu Gln Leu Val Val Asp Val Asp Gly Asn Ile
            100                 105                 110
Val Asn Glu Val Lys Leu Leu Arg Lys Glu Ser Arg Asn Met Asn Ser
        115                 120                 125
Arg Val Thr Gln Leu Tyr Met Gln Leu Leu His Glu Ile Ile Arg Lys
    130                 135                 140
Arg Asp Asn Ser Leu Glu Leu Ser Gln Leu Glu Asn Lys Ile Leu Asn
145                 150                 155                 160
Val Thr Thr Glu Met Leu Lys Met Ala Thr Arg Tyr Arg Glu Leu Glu
                165                 170                 175
Val Lys Tyr Ala Ser Leu Thr Asp Leu Val Asn Asn Gln Ser Val Met
            180                 185                 190
Ile Thr Leu Leu Glu Glu Gln Cys Leu Arg Ile Phe Ser Arg Gln Asp
        195                 200                 205

Thr His Val Ser Pro Pro Leu Val Gln Val Val Pro Gln His Ile Pro
    210                 215                 220

Asn Ser Gln Gln Tyr Thr Pro Gly Leu Leu Gly Gly Asn Glu Ile Gln
225                 230                 235                 240

Arg Asp Pro Gly Tyr Pro Arg Asp Leu Met Pro Pro Pro Asp Leu Ala
                245                 250                 255

Thr Ser Pro Thr Lys Ser Pro Phe Lys Ile Pro Pro Val Thr Phe Ile
            260                 265                 270

Asn Glu Gly Pro Phe Lys Asp Cys Gln Gln Ala Lys Glu Ala Gly His
        275                 280                 285

Ser Val Ser Gly Ile Tyr Met Ile Lys Pro Glu Asn Ser Asn Gly Pro
    290                 295                 300

Met Gln Leu Trp Cys Glu Asn Ser Leu Asp Pro Gly Gly Trp Thr Val
305                 310                 315                 320

Ile Gln Lys Arg Thr Asp Gly Ser Val Asn Phe Phe Arg Asn Trp Glu
                325                 330                 335

Asn Tyr Lys Lys Gly Phe Gly Asn Ile Asp Gly Glu Tyr Trp Leu Gly
            340                 345                 350

Leu Glu Asn Ile Tyr Met Leu Ser Asn Gln Asp Asn Tyr Lys Leu Leu
        355                 360                 365

Ile Glu Leu Glu Asp Trp Ser Asp Lys Lys Val Tyr Ala Glu Tyr Ser
    370                 375                 380

Ser Phe Arg Leu Glu Pro Glu Ser Glu Phe Tyr Arg Leu Arg Leu Gly
385                 390                 395                 400

Thr Tyr Gln Gly Asn Ala Gly Asp Ser Met Met Trp His Asn Gly Lys
                405                 410                 415

Gln Phe Thr Thr Leu Asp Arg Asp Lys Asp Met Tyr Ala Gly Asn Cys
            420                 425                 430

Ala His Phe His Lys Gly Gly Trp Trp Tyr Asn Ala Cys Ala His Ser
        435                 440                 445

Asn Leu Asn Gly Val Trp Tyr Arg Gly Gly His Tyr Arg Ser Lys His
    450                 455                 460

Gln Asp Gly Ile Phe Trp Ala Glu Tyr Arg Gly Gly Ser Tyr Ser Leu
465                 470                 475                 480

Arg Ala Val Gln Met Met Ile Lys Pro Ile Asp
                485                 490

<210> 3
<211> 491
<212> PRT
<213> Artificial Sequence

<220>
<223> Description of Artificial Sequence: Scarface 2
      analog

<220>

<223> Xaa at position 277 is Pro, Phe, or Trp

<220>
<223> Xaa at position 278 is Ala, Lys, or Arg

<220>
<223> Xaa at position 279 is Glu, or Asp

<220>
<223> Xaa at position 281 is Thr, Ala, Gln, or Leu

<220>
<223> Xaa at position 283 is Ile, Leu, Ala, or Val

<220>
<223> Xaa at position 284 is Tyr, Phe, Leu, or Lys

<220>
<223> Xaa at position 285 is Asn, Gln, Lys, or Glu

<220>
<223> Xaa at position 286 is Arg, Ala, Val, Ser, or Asp

<220>
<223> Xaa at position 288 is Glu, His, or Phe

<220>
<223> Xaa at position 289 is His, Arg, Ser, Asn, Thr, or
      Asp

<220>
<223> Xaa at position 290 is Thr, Lys, Val, or Gln

<220>

<223> Xaa at position 304 is Val, Pro, Leu, or  Glu

<220>

<223> Xaa at position 305 is Phe, Met, Ile, or Lys

<220>

<223> Xaa at position 306 is His, Lys, Gln, or Leu

<220>

<223> Xaa at position 307 is Val, Leu, or Ala

<220>

<223> Xaa at position 308 is Tyr, Phe, Trp, or Asn

<220>

<223> Xaa at position 310 is Asp, Asn, Glu, or Lys

<220>

<223> Xaa at position 311 is Val, Met, Asn, or Gln

<220>

<223> Xaa at position 312 is Ile, Thr, Ser, Arg, Glu, or
      Asp

<220>

<223> Xaa at position 313 is Ser, Leu, His, Ala, or Val

<220>

<223> Xaa at position 314 is Asp, Gly, or Asn

<220>

<223> Xaa at position 315 is Gly, Pro, or Asp

<220>

<223> Xaa at position 316 is Ser, or Gly

<220>

<223> Xaa at position 317 is Pro, or Gly

<220>

<223> Xaa at position 320 is Leu, Val, or Ile

<220>

<223> Xaa at position 323 is His, Arg, or Lys

<220>

<223> Xaa at position 325 is Ile, Glu, Leu, Thr, or His

<220>

<223> Xaa at position 329 is Gln, Val, or Leu

<220>

<223> Xaa at position 330 is Asn, or Asp

<220>

<223> Xaa at position 332 is Asn, Phe, or Gln

<220>

<223> Xaa at position 333 is Glu, or Arg

<220>

<223> Xaa at position 334 is Thr, Gly, Asn, or Pro

<220>

<223> Xaa at position 336 is Glu, or Lys

<220>

<223> Xaa at position 337 is Asn, Glu, Thr, or Ala

<220>

<223> Xaa at position 340 is Tyr, Met, Val, Gln, Lys, or
      Ala

<220>

<223> Xaa at position 344 is Arg, Asn, or Asp

<220>

<223> Xaa at position 345 is Leu, Pro, or Ile

<220>

<223> Xaa at position 346 is Asp, Ser, or His

<220>

<223> Xaa at position 349 is Phe, or Tyr

<220>

<223> Xaa at position 353 is Leu, or Asn

<220>

<223> Xaa at position 355 is Lys, Asn, or Phe

<220>

<223> Xaa at position 356 is Ile, or Val

<220>

<223> Xaa at position 357 is Tyr, His, Ser, or Phe

<220>

<223> Xaa at position 358 is Ser, Met, Trp, Gln, or Ala

<220>
<223> Xaa at position 359 is Ile, or Leu

<220>
<223> Xaa at position 360 is Val, Thr, Ser, or Met

<220>
<223> Xaa at position 361 is Lys, Gly, Asn, or Ser

<220>
<223> Xaa at position 362 is Gln, or Asp

<220>
<223> Xaa at position 363 is Ser, Arg, Asp, Gly, or Gln

<220>
<223> Xaa at position 364 is Asn, Arg, or Gln

<220>

<400> 3
Met Lys Thr Phe Thr Trp Thr Leu Gly Val Leu Phe Phe Leu Leu Val
1               5                   10                  15

Asp Thr Gly His Cys Arg Gly Gly Gln Phe Lys Ile Lys Lys Ile Asn
            20                  25                  30

Gln Arg Arg Tyr Pro Arg Ala Thr Asp Gly Lys Glu Glu Ala Lys Lys
        35                  40                  45

Cys Ala Tyr Thr Phe Leu Val Pro Glu Gln Arg Ile Thr Gly Pro Ile

50                  55                  60

Cys Val Asn Thr Lys Gly Gln Asp Ala Ser Thr Ile Lys Asp Met Ile
65                  70                  75                  80

Thr Arg Met Asp Leu Glu Asn Leu Lys Asp Val Leu Ser Arg Gln Lys
                85                  90                  95

Arg Glu Ile Asp Val Leu Gln Leu Val Val Asp Val Asp Gly Asn Ile
            100                 105                 110

Val Asn Glu Val Lys Leu Leu Arg Lys Glu Ser Arg Asn Met Asn Ser
        115                 120                 125

Arg Val Thr Gln Leu Tyr Met Gln Leu Leu His Glu Ile Ile Arg Lys
    130                 135                 140

Arg Asp Asn Ser Leu Glu Leu Ser Gln Leu Glu Asn Lys Ile Leu Asn
145                 150                 155                 160

Val Thr Thr Glu Met Leu Lys Met Ala Thr Arg Tyr Arg Glu Leu Glu
                165                 170                 175

Val Lys Tyr Ala Ser Leu Thr Asp Leu Val Asn Asn Gln Ser Val Met
            180                 185                 190

Ile Thr Leu Leu Glu Glu Gln Cys Leu Arg Ile Phe Ser Arg Gln Asp
        195                 200                 205

Thr His Val Ser Pro Pro Leu Val Gln Val Val Pro Gln His Ile Pro
    210                 215                 220

Asn Ser Gln Gln Tyr Thr Pro Gly Leu Leu Gly Gly Asn Glu Ile Gln

225                 230                 235                 240

Arg Asp Pro Gly Tyr Pro Arg Asp Leu Met Pro Pro Pro Asp Leu Ala
                245                 250                 255

Thr Ser Pro Thr Lys Ser Pro Phe Lys Ile Pro Pro Val Thr Phe Ile
            260                 265                 270

Asn Glu Gly Pro Xaa Xaa Xaa Cys Xaa Xaa Xaa Xaa Xaa Xaa Gly Xaa
        275                 280                 285

Xaa Xaa Xaa Xaa Xaa Xaa Xaa Xaa Xaa Xaa Xaa Xaa Xaa Xaa Xaa Xaa
    290                 295                 300

Xaa Xaa Xaa Xaa Cys Xaa Xaa Xaa Xaa Xaa Xaa Xaa Xaa Trp Thr Xaa
305                 310                 315                 320

Ile Gln Xaa Arg Xaa Asp Gly Ser Xaa Xaa Phe Xaa Xaa Xaa Trp Xaa
                325                 330                 335

Xaa Tyr Lys Xaa Gly Phe Gly Xaa Xaa Xaa Gly Glu Xaa Trp Leu Gly
            340                 345                 350

Xaa Glu Xaa Xaa Xaa Xaa Xaa Xaa Xaa Xaa Xaa Xaa Xaa Xaa Xaa Xaa
        355                 360                 365

Xaa Xaa Xaa Xaa Asp Trp Xaa Xaa Xaa Xaa Xaa Xaa Xaa Xaa Xaa Xaa
    370                 375                 380

Xaa Xaa Xaa Xaa Xaa Xaa Xaa Xaa Xaa Xaa Tyr Xaa Xaa Xaa Leu Xaa
385                 390                 395                 400

Xaa Xaa Xaa Gly Xaa Xaa Xaa Xaa Xaa Xaa Xaa Xaa Xaa Xaa Xaa Xaa

                405                 410                 415

Xaa Phe Xaa Thr Xaa Xaa Xaa Xaa Xaa Xaa Xaa Xaa Xaa Xaa Xaa Cys

            420                 425                 430

Xaa Xaa Xaa Xaa Xaa Gly Gly Trp Trp Xaa Xaa Xaa Cys Xaa Xaa Xaa

        435                 440                 445

Asn Leu Asn Gly Xaa Xaa Xaa Xaa Xaa Xaa Xaa Xaa Xaa Xaa Lys His

    450                 455                 460

Gln Asp Gly Ile Phe Trp Ala Glu Tyr Arg Gly Gly Ser Tyr Ser Leu

465                 470                 475                 480

Arg Ala Val Gln Met Met Ile Lys Pro Ile Asp

                485                 490

SEQUENCE LISTING

<110> Burgett, Stanley G.
      Leonard, Rebecca A.
      Rosteck Jr., Paul R.
      Sankhavaram, Patanjali R.

<120> Angiopoietin Related Gene Sequence Scarface 2

<130> X-12292

<140>
<141>

<160> 3

<170> PatentIn Ver. 2.0

<210> 1
<211> 2393
<212> DNA
<213> Homo sapiens

<220>
<221> CDS
<222> (357)..(1829)

<400> 1

atacaatctg ggtcag[?][?]gc agctggttac tgcatttctc catgtggcag acagagcaaa 60

gccacaacgc tttctctgct ggattaaaga cggcccacag accagaactt ccactatact 120

acttaaaatt acataggtgg cttgtcaaat tcaattgatt agtattgtaa aaggaaaaag 180

aagttccttc ttacagcttg gattcaacgg tccaaaacaa aaatgcagct gccattaaag 240

tcacagatga acaaacttct acactgattt ttaaaatcaa gaataagggc agcaagtttc 300

tggattcact gaatcaacag acacaaaaag acatcatttt acaacctcat ttcaaa atg 359
Met
1

aag act ttt acc tgg acc cta ggt gtg cta ttc ttc cta cta gtg gac 407
Lys Thr Phe Thr Trp Thr Leu Gly Val Leu Phe Phe Leu Leu Val Asp
            5                   10                  15

act gga cat tgc aga ggt gga caa ttc aaa att aaa aaa ata aac cag 455
Thr Gly His Cys Arg Gly Gly Gln Phe Lys Ile Lys Lys Ile Asn Gln
        20                  25                  30

aga aga tac cct cgt gcc aca gat ggt aaa gag gaa gca aag aaa tgt 503
Arg Arg Tyr Pro Arg Ala Thr Asp Gly Lys Glu Glu Ala Lys Lys Cys
    35                  40                  45

gca tac aca ttc ctg gta cct gaa caa aga ata aca ggg cca atc tgt 551
Ala Tyr Thr Phe Leu Val Pro Glu Gln Arg Ile Thr Gly Pro Ile Cys

50                  55                  60                  65

gtc aac acc aag ggg caa gat gca agt acc att aaa gac atg atc acc 599
Val Asn Thr Lys Gly Gln Asp Ala Ser Thr Ile Lys Asp Met Ile Thr
                70                  75                  80

agg atg gac ctt gaa aac ctg aag gat gtg ctc tcc agg cag aag cgg 647
Arg Met Asp Leu Glu Asn Leu Lys Asp Val Leu Ser Arg Gln Lys Arg
            85                  90                  95

gag ata gat gtt ctg caa ctg gtg gtg gat gta gat gga aac att gtg 695
Glu Ile Asp Val Leu Gln Leu Val Val Asp Val Asp Gly Asn Ile Val
        100                 105                 110

aat gag gta aag ctg ctg aga aag gaa agc cgt aac atg aac tct cgt 743
Asn Glu Val Lys Leu Leu Arg Lys Glu Ser Arg Asn Met Asn Ser Arg
    115                 120                 125

gtt act caa ctc tat atg caa tta tta cat gag att atc cgt aag agg 791
Val Thr Gln Leu Tyr Met Gln Leu Leu His Glu Ile Ile Arg Lys Arg
130                 135                 140                 145

gat aat tca ctt gaa ctt tcc caa ctg gaa aac aaa atc ctc aat gtc 839
Asp Asn Ser Leu Glu Leu Ser Gln Leu Glu Asn Lys Ile Leu Asn Val
                150                 155                 160

acc aca gaa atg ttg aag atg gca aca aga tac agg gaa cta gag gtg 887
Thr Thr Glu Met Leu Lys Met Ala Thr Arg Tyr Arg Glu Leu Glu Val

            165                 170                 175

aaa tac gct tcc ttg act gat ctt gtc aat aac caa tct gtg atg atc 935
Lys Tyr Ala Ser Leu Thr Asp Leu Val Asn Asn Gln Ser Val Met Ile
        180                 185                 190

act ttg ttg gaa gaa cag tgc ttg agg ata ttt tcc cga caa gac acc 983
Thr Leu Leu Glu Glu Gln Cys Leu Arg Ile Phe Ser Arg Gln Asp Thr
    195                 200                 205

cat gtg tct ccc cca ctt gtc cag gtg gtg cca caa cat att cct aac 1031
His Val Ser Pro Pro Leu Val Gln Val Val Pro Gln His Ile Pro Asn
210                 215                 220                 225

agc caa cag tat act cct ggt ctg ctg gga ggt aac gag att cag agg 1079
Ser Gln Gln Tyr Thr Pro Gly Leu Leu Gly Gly Asn Glu Ile Gln Arg
                230                 235                 240

gat cca ggt tac ccc aga gat tta atg cca cca cct gat ctg gca act 1127
Asp Pro Gly Tyr Pro Arg Asp Leu Met Pro Pro Pro Asp Leu Ala Thr
            245                 250                 255

tct ccc acc aaa agc cct ttc aag ata cca ccg gta act ttc atc aat 1175
Ser Pro Thr Lys Ser Pro Phe Lys Ile Pro Pro Val Thr Phe Ile Asn
        260                 265                 270

gaa gga cca ttc aaa gac tgt cag caa gca aaa gaa gct ggg cat tcg 1223
Glu Gly Pro Phe Lys Asp Cys Gln Gln Ala Lys Glu Ala Gly His Ser

    275                 280                 285

gtc agt ggg att tat atg att aaa cct gaa aac agc aat gga cca atg 1271

Val Ser Gly Ile Tyr Met Ile Lys Pro Glu Asn Ser Asn Gly Pro Met

290                 295                 300                 305

cag tta tgg tgt gaa aac agt ttg gac cct ggg ggt tgg act gtt att 1319

Gln Leu Trp Cys Glu Asn Ser Leu Asp Pro Gly Gly Trp Thr Val Ile

                310                 315                 320

cag aaa aga aca gac ggc tct gtc aac ttc ttc aga aat tgg gaa aat 1367

Gln Lys Arg Thr Asp Gly Ser Val Asn Phe Phe Arg Asn Trp Glu Asn

            325                 330                 335

tat aag aaa ggg ttt gga aac att gac gga gaa tac tgg ctt gga ctg 1415

Tyr Lys Lys Gly Phe Gly Asn Ile Asp Gly Glu Tyr Trp Leu Gly Leu

        340                 345                 350

gaa aat atc tat atg ctt agc aat caa gat aat tac aag tta ttg att 1463

Glu Asn Ile Tyr Met Leu Ser Asn Gln Asp Asn Tyr Lys Leu Leu Ile

    355                 360                 365

gaa tta gaa gac tgg agt gat aaa aaa gtc tat gca gaa tac agc agc 1511

Glu Leu Glu Asp Trp Ser Asp Lys Lys Val Tyr Ala Glu Tyr Ser Ser

370                 375                 380                 385

ttt cgt ctg gaa cct gaa agt gaa ttc tat aga ctg cgc ctg gga act 1559

Phe Arg Leu Glu Pro Glu Ser Glu Phe Tyr Arg Leu Arg Leu Gly Thr

                390                 395                 400
"
"
tac cag gga aat gca ggg gat tct atg atg tgg cat aat ggt aaa caa 1607
"
Tyr Gln Gly Asn Ala Gly Asp Ser Met Met Trp His Asn Gly Lys Gln
"
405 410 415
"
"
ttc acc aca ctg gac aga gat aaa gat atg tat gca gga aac tgc gcc 1655
"
Phe Thr Thr Leu Asp Arg Asp Lys Asp Met Tyr Ala Gly Asn Cys Ala
"
420 425 430
"
"
cac ttt cat aaa gga ggc tgg tgg tac aat gcc tgt gca cat tct aac 1703
"
His Phe His Lys Gly Gly Trp Trp Tyr Asn Ala Cys Ala His Ser Asn
"
435 440 445
"
"
cta aat gga gta tgg tac aga gga ggc cat tac aga agc aag cac caa 1751
"
Leu Asn Gly Val Trp Tyr Arg Gly Gly His Tyr Arg Ser Lys His Gln
"
450 455 460 465
"
"
gat gga att ttc tgg gcc gaa tac aga ggc ggg tca tac tcc tta aga 1799
"
Asp Gly Ile Phe Trp Ala Glu Tyr Arg Gly Gly Ser Tyr Ser Leu Arg
"
470 475 480
"
"
gca gtt cag atg atg atc aag cct att gac tgaagagaga cactcgccaa 1849
"
Ala Val Gln Met Met Ile Lys Pro Ile Asp
"
485 490
"
"
tttaaatgac acagaacttt gtacttttca gctcttaaaa atgtaaatgt tacatgtata 1909
"
"

ttacttggca caatttattt ctacacataa agtttttaaa atgaatttta ccgtaactat 1969

aaaagggaac ctataaatgt agtttcatct gtcgtcaatt actgcagaaa attatgtgta 2029

tccacaacct agttatttta aaaattatgt tgactaaata caaagtttgt tttctaaaat 2089

gtaaatattt gccacaatgt aaagcaaatc ttagctatat tttaaatcat aaataacatg 2149

ttcaagatac ttaacaattt atttaaaatc taagattgct ctaacgtcta gtgaaaaaaa 2209

tatttttaaa atttcagcca aataatgcat tttatttata aaaatacaga cagaaaatta 2269

gggagaaacc tctagttttg ccaatagaaa atgcttcttc cattgaataa aagttatttc 2329

aaattgaaaa aaaaaaaaaa aaaaaaaaaa aaaaaaaaaa aaaaaaaaaa aaaaaaaaaa 2389

aaaa 2393

<210> 2

<211> 491

<212> PRT

<213> Homo sapiens

<400> 2

Met Lys Thr Phe Thr Trp Thr Leu Gly Val Leu Phe Phe Leu Leu Val
1               5                   10                  15

Asp Thr Gly His Cys Arg Gly Gly Gln Phe Lys Ile Lys Lys Ile Asn
"
20 25 30
"

"
Gln Arg Arg Tyr Pro Arg Ala Thr Asp Gly Lys Glu Glu Ala Lys Lys
"
35 40 45
"

"
Cys Ala Tyr Thr Phe Leu Val Pro Glu Gln Arg Ile Thr Gly Pro Ile
"
50 55 60
"

"
Cys Val Asn Thr Lys Gly Gln Asp Ala Ser Thr Ile Lys Asp Met Ile
"
65 70 75 80
"

"
Thr Arg Met Asp Leu Glu Asn Leu Lys Asp Val Leu Ser Arg Gln Lys
"
85 90 95
"

"
Arg Glu Ile Asp Val Leu Gln Leu Val Val Asp Val Asp Gly Asn Ile
"
100 105 110
"

"
Val Asn Glu Val Lys Leu Leu Arg Lys Glu Ser Arg Asn Met Asn Ser
"
115 120 125
"

"
Arg Val Thr Gln Leu Tyr Met Gln Leu Leu His Glu Ile Ile Arg Lys
"
130 135 140
"

"
Arg Asp Asn Ser Leu Glu Leu Ser Gln Leu Glu Asn Lys Ile Leu Asn
"
145 150 155 160
"

"
Val Thr Thr Glu Met Leu Lys Met Ala Thr Arg Tyr Arg Glu Leu Glu
"

165 170 175
Val Lys Tyr Ala Ser Leu Thr Asp Leu Val Asn Asn Gln Ser Val Met
180 185 190
Ile Thr Leu Leu Glu Glu Gln Cys Leu Arg Ile Phe Ser Arg Gln Asp
195 200 205
Thr His Val Ser Pro Pro Leu Val Gln Val Val Pro Gln His Ile Pro
210 215 220
Asn Ser Gln Gln Tyr Thr Pro Gly Leu Leu Gly Gly Asn Glu Ile Gln
225 230 235 240
Arg Asp Pro Gly Tyr Pro Arg Asp Leu Met Pro Pro Pro Asp Leu Ala
245 250 255
Thr Ser Pro Thr Lys Ser Pro Phe Lys Ile Pro Pro Val Thr Phe Ile
260 265 270
Asn Glu Gly Pro Phe Lys Asp Cys Gln Gln Ala Lys Glu Ala Gly His
275 280 285
Ser Val Ser Gly Ile Tyr Met Ile Lys Pro Glu Asn Ser Asn Gly Pro
290 295 300
Met Gln Leu Trp Cys Glu Asn Ser Leu Asp Pro Gly Gly Trp Thr Val
305 310 315 320

Ile Gln Lys Arg Thr Asp Gly Ser Val Asn Phe Phe Arg Asn Trp Glu
325 330 335
Asn Tyr Lys Lys Gly Phe Gly Asn Ile Asp Gly Glu Tyr Trp Leu Gly
340 345 350
Leu Glu Asn Ile Tyr Met Leu Ser Asn Gln Asp Asn Tyr Lys Leu Leu
355 360 365
Ile Glu Leu Glu Asp Trp Ser Asp Lys Lys Val Tyr Ala Glu Tyr Ser
370 375 380
Ser Phe Arg Leu Glu Pro Glu Ser Glu Phe Tyr Arg Leu Arg Leu Gly
385 390 395 400
Thr Tyr Gln Gly Asn Ala Gly Asp Ser Met Met Trp His Asn Gly Lys
405 410 415
Gln Phe Thr Thr Leu Asp Arg Asp Lys Asp Met Tyr Ala Gly Asn Cys
420 425 430
Ala His Phe His Lys Gly Gly Trp Trp Tyr Asn Ala Cys Ala His Ser
435 440 445
Asn Leu Asn Gly Val Trp Tyr Arg Gly Gly His Tyr Arg Ser Lys His
450 455 460

Gln Asp Gly Ile Phe Trp Ala Glu Tyr Arg Gly Gly Ser Tyr Ser Leu
465 470 475 480
Arg Ala Val Gln Met Met Ile Lys Pro Ile Asp
485 490
<210> 3
<211> 491
<212> PRT
<213> Artificial Sequence
<220>
<223> Description of Artificial Sequence: Scarface 2
analog
<220>
<223> Xaa at position 277 is Pro, Phe, or Trp
<220>
<223> Xaa at position 278 is Ala, Lys, or Arg
<220>
<223> Xaa at position 279 is Glu, or Asp
<220>
<223> Xaa at position 281 is Thr, Ala, Gln, or Leu

<220>
″
<223> Xaa at position 283 is Ile, Leu, Ala, or Val
″

″
<220>
″
<223> Xaa at position 284 is Tyr, Phe, Leu, or Lys
″

″
<220>
″
<223> Xaa at position 285 is Asn, Gln, Lys, or Glu
″

″
<220>
″
<223> Xaa at position 286 is Arg, Ala, Val, Ser, or Asp
″

″
<220>
″
<223> Xaa at position 288 is Glu, His, or Phe
″

″
<220>
″
<223> Xaa at position 289 is His, Arg, Ser, Asn, Thr, or
″
      Asp
″

″
<220>
″
<223> Xaa at position 290 is Thr, Lys, Val, or Gln
″

″
<220>
″
<223> Xaa at position 304 is Val, Pro, Leu, or  Glu
″

″
<220>
″
<223> Xaa at position 305 is Phe, Met, Ile, or Lys
″

″

<220>
"
<223> Xaa at position 306 is His, Lys, Gln, or Leu
"

"
<220>
"
<223> Xaa at position 307 is Val, Leu, or Ala
"

"
<220>
"
<223> Xaa at position 308 is Tyr, Phe, Trp, or Asn
"

"
<220>
"
<223> Xaa at position 310 is Asp, Asn, Glu, or Lys
"

"
<220>
"
<223> Xaa at position 311 is Val, Met, Asn, or Gln
"

"
<220>
"
<223> Xaa at position 312 is Ile, Thr, Ser, Arg, Glu, or
"
      Asp
"

"
<220>
"
<223> Xaa at position 313 is Ser, Leu, His, Ala, or Val
"

"
<220>
"
<223> Xaa at position 314 is Asp, Gly, or Asn
"

"
<220>
"
<223> Xaa at position 315 is Gly, Pro, or Asp
"

"

<220>
"
<223> Xaa at position 316 is Ser, or Gly
"

"
<220>
"
<223> Xaa at position 317 is Pro, or Gly
"

"
<220>
"
<223> Xaa at position 320 is Leu, Val, or Ile
"

"
<220>
"
<223> Xaa at position 323 is His, Arg, or Lys
"

"
<220>
"
<223> Xaa at position 325 is Ile, Glu, Leu, Thr, or His
"

"
<220>
"
<223> Xaa at position 329 is Gln, Val, or Leu
"

"
<220>
"
<223> Xaa at position 330 is Asn, or Asp
"

"
<220>
"
<223> Xaa at position 332 is Asn, Phe, or Gln
"

"
<220>
"
<223> Xaa at position 333 is Glu, or Arg
"

"
<220>
"

<223> Xaa at position 334 is Thr, Gly, Asn, or Pro
"

"
<220>
"
<223> Xaa at position 336 is Glu, or Lys
"

"
<220>
"
<223> Xaa at position 337 is Asn, Glu, Thr, or Ala
"

"
<220>
"
<223> Xaa at position 340 is Tyr, Met, Val, Gln, Lys, or
"
      Ala
"

"
<220>
"
<223> Xaa at position 344 is Arg, Asn, or Asp
"

"
<220>
"
<223> Xaa at position 345 is Leu, Pro, or Ile
"

"
<220>
"
<223> Xaa at position 346 is Asp, Ser, or His
"

"
<220>
"
<223> Xaa at position 349 is Phe, or Tyr
"

"
<220>
"
<223> Xaa at position 353 is Leu, or Asn
"

"
<220>
"

<223> Xaa at position 355 is Lys, Asn, or Phe
"

"
<220>
"
<223> Xaa at position 356 is Ile, or Val
"

"
<220>
"
<223> Xaa at position 357 is Tyr, His, Ser, or Phe
"

"
<220>
"
<223> Xaa at position 358 is Ser, Met, Trp, Gln, or Ala
"

"
<220>
"
<223> Xaa at position 359 is Ile, or Leu
"

"
<220>
"
<223> Xaa at position 360 is Val, Thr, Ser, or Met
"

"
<220>
"
<223> Xaa at position 361 is Lys, Gly, Asn, or Ser
"

"
<220>
"
<223> Xaa at position 362 is Gln, or Asp
"

"
<220>
"
<223> Xaa at position 363 is Ser, Arg, Asp, Gly, or Gln
"

"
<220>
"
<223> Xaa at position 364 is Asn, Arg, or Gln
"

″
<220>
″
<223> Xaa at position 365 is Tyr, or Ser
″

″
<220>
″
<223> Xaa at position 366 is Val, Met, Lys, or Arg
″

″
<220>
″
<223> Xaa at position 368 is Arg, Leu, Ala, or Lys
″

″
<220>
″
<223> Xaa at position 369 is Ile, or Val
″

″
<223> Xaa at position 370 is Glu, His, Gln, or Thr
″

″
<223> Xaa at position 371 is Leu, or Met
″

″
<223> Xaa at position 372 is Glu, Arg, Lys, or Met
″

″
<223> Xaa at position 375 is Lys, Ser, Glu, or Asp
″

″
<223> Xaa at position 376 is Asp, or Gly
″

″
<223> Xaa at position 377 is Asn, Lys, or Arg
″

″
<223> Xaa at position 378 is Lys, Ala, Glu, or Arg
″

″
<223> Xaa at position 379 is His, Glu, Val, or Ala
″

"
<223> Xaa at position 380 is Tyr, Phe, or Leu
"

"
<223> Xaa at position 381 is Ile, Leu, Ala, or Ser
"

"
<223> Xaa at position 382 is Glu, Leu, or Gln
"

"
<223> Xaa at position 383 is Tyr, or Phe
"

"
<223> Xaa at position 384 is Ser, Ala, Asp, or Glu
"

"
<223> Xaa at position 385 is Ser, His, or Arg
"

"
<223> Xaa at position 386 is Phe, or Val
"

"
<223> Xaa at position 387 is Tyr, His, or Arg
"

"
<223> Xaa at position 388 is Leu, or Ile
"

"
<223> Xaa at position 389 is Gly, Glu, or Ser
"

"
<223> Xaa at position 390 is Asn, Pro, Ser, or Gly
"

"
<223> Xaa at position 391 is His, or Glu
"

"
<223> Xaa at position 392 is Glu, Asp, Ser, or Lys
"

"
<223> Xaa at position 393 is Thr, Gln, Leu, or Glu
"

"
<223> Xaa at position 394 is Asn, Tyr, Phe or Ala
"

"
<223> Xaa at position 396 is Thr, Ser, Arg, or Lys
"

"
<223> Xaa at position 397 is Leu, or Ile
"

"
<223> Xaa at position 398 is His, Arg, Gln, or Tyr
"

"
<223> Xaa at position 400 is Val, Ala, Gly, or Lys
"

"
<223> Xaa at position 401 is Ala, Gly, Arg, Thr, or Pro
"

"
<223> Xaa at position 402 is Ile, Val, Tyr, Leu, or His
"

"
<223> Xaa at position 403 is Thr, Ala, Gln, or His
"

"
<223> Xaa at position 405 is Asn, Thr, or Gln
"

"
<223> Xaa at position 406 is Val, Ala, or Leu
"

"
<223> Xaa at position 407 is Pro, or Gly
"

"
<223> Xaa at position 408 is Asn, Asp, Ser, or Thr
"

"
<223> Xaa at position 409 is Ala, Ser, or Val
"

"
<223> Xaa at position 410 is Ile, Leu, Phe, Met, or Pro
"

"
<223> Xaa at position 411 is Pro, Ile, Ser, Thr, or Met
"

"
<223> Xaa at position 412 is Glu, Trp, Ser, Leu, or Gln
"

"
<223> Xaa at position 413 is Asn, His, Pro, or Gly
"

"
<223> Xaa at position 414 is Lys, Asn, or Leu
"

"
<223> Xaa at position 415 Asp, Gly, or Ser
"

"
<223> Xaa at position 416 is Leu, Asn, Lys, Ala, or Val
"

"
<223> Xaa at position 417 is Val, Gln, Asp, or Pro
"

"
<223> Xaa at position 419 is Ser, or Thr
"

"
<223> Xaa at position 421 is Trp, Lys, or Leu
"

"
<223> Xaa at position 422 is His, or Asp
"

"
<223> Xaa at position 423 is Arg, Gly, Ala, or Gln
"

"
<223> Xaa at position 424 is His, or Asp
"

"
<223> Xaa at position 425 is Lys, Asn, or His,
"

"
<223> Xaa at position 426 is Ala, or Asp
"

"
<223> Xaa at positio[?][?] 427 is Lys, Asn, Val, Met or Leu
"

"
<223> Xaa at position 428 is Gly, Arg, Tyr, or Cys
"

"
<223> Xaa at position 429 is His, Met, Ile, Thr, Ala, or Asp
"

"
<223> Xaa at position 430 is Phe, Cys, Gly, or Lys
"

"
<223> Xaa at p[?][?][?][?][?][?]n 431 is Asn, or Lys
"

"
<223> Xaa at positio[?] 4[?]3 is Pro, Ala, or Ser
"

"
<223> Xaa at position 434 is Glu, His, Leu, Gln, or Lys
"

"
<223> Xaa at position 435 is Gly, Ser, Phe, Tyr, or Met
"

"
<223> Xaa at position 436 is Tyr, Leu, Gln, or His
"

"
<223> Xaa at position 437 is Ser, Lys, or Thr
"

"
<223> Xaa at pos[?][?]i[?]n 442 is His, Phe, or Tyr
"

"
<223> Xaa at position 443 is Asp, Asn, or Gly
"

"
<223> Xaa at position 444 is Glu, Ala, or Thr
"

"
<223> Xaa at position 446 is Gly, Ala, or Ser
"

<223> Xaa at position 447 is Glu, His, or Pro

<223> Xaa at position 448 is Asn, or Ser

<223> Xaa at position 453 is Lys, Gln, Met, or Val

<223> Xaa at position 454 is Tyr, Trp, or Phe

<223> Xaa at position 455 is Asn, Phe, or Tyr

<223> Xaa at position 456 is Lys, Arg, Pro, or Thr

<223> Xaa at position 457 is Pro, Ser, Gly, Gln, or Ala

<223> Xaa at position 458 is Arg, Ile, Gly, or Arg

<223> Xaa at position 459 is Ala, Gln, or His

<223> Xaa at position 460 is Lys, Tyr, Asn, or Gln

<223> Xaa at position 461 is Ser, Arg, Thr, or His

<223> Xaa at position 462 is Lys, Gln, Ser, Asn, or Gly

<400> 3

Met Lys Thr Phe Thr Trp Thr Leu Gly Val Leu Phe Phe Leu Leu Val
1               5                   10                  15

"
Asp Thr Gly His Cys Arg Gly Gly Gln Phe Lys Ile Lys Lys Ile Asn
"
            2[?]                    25                    30
"

"
Gln Arg Arg Tyr Pro Arg Ala Thr Asp Gly Lys Glu Glu Ala Lys Lys
"
        35                    40                    45
"

"
Cys Ala Tyr Thr Phe Leu Val Pro Glu Gln Arg Ile Thr Gly Pro Ile
"
    50                    55                    60
"

"
Cys Val Asn Thr Lys Gly Gln Asp Ala Ser Thr Ile Lys Asp Met Ile
"
65                    [?]0                    75                    80
"

"
Thr Arg Met Asp [?]eu Glu Asn Leu Lys Asp Val Leu Ser Arg Gln Lys
"
                [?]5                    90                    95
"

"
Arg Glu Ile Asp Val Leu Gln Leu Val Val Asp Val Asp Gly Asn Ile
"
            100                    105                    110
"

"
Val Asn Glu Val Lys Leu Leu Arg Lys Glu Ser Arg Asn Met Asn Ser
"
        115                    120                    125
"

"
Arg Val Thr Gln Leu Tyr Met Gln Leu Leu His Glu Ile Ile Arg Lys
"
    130                    135                    140
"

"
Arg Asp Asn Ser Leu Glu Leu Ser Gln Leu Glu Asn Lys Ile Leu Asn
"
145                    150                    155                    160
"

"

Val Thr Thr Glu Met Leu Lys Met Ala Thr Arg Tyr Arg Glu Leu Glu
"
165 170 175
"

"
Val Lys Tyr Ala Ser Leu Thr Asp Leu Val Asn Asn Gln Ser Val Met
"
180 185 190
"

"
Ile Thr Leu Leu Glu Glu Gln Cys Leu Arg Ile Phe Ser Arg Gln Asp
"
195 200 205
"

"
Thr His Val Ser Pro Pro Leu Val Gln Val Val Pro Gln His Ile Pro
"
210 215 220
"

"
Asn Ser Gln Gln Tyr Thr Pro Gly Leu Leu Gly Gly Asn Glu Ile Gln
"
225 230 235 240
"

"
Arg Asp Pro Gly Tyr Pro Arg Asp Leu Met Pro Pro Pro Asp Leu Ala
"
245 250 255
"

"
Thr Ser Pro Thr Lys Ser Pro Phe Lys Ile Pro Pro Val Thr Phe Ile
"
260 265 270
"

"
Asn Glu Gly Pro Xaa Xaa Xaa Cys Xaa Xaa Xaa Xaa Xaa Xaa Gly Xaa
"
275 280 285
"

"
Xaa Xaa Xaa Xaa Xaa Xaa Xaa Xaa Xaa Xaa Xaa Xaa Xaa Xaa Xaa Xaa
"
290 295 300
"

"
Xaa Xaa Xaa Xaa Cys Xaa Xaa Xaa Xaa Xaa Xaa Xaa Xaa Trp Thr Xaa
"

305                 310                 315                 320

Ile Gln Xaa Arg Xaa Asp Gly Ser Xaa Xaa Phe Xaa Xaa Xaa Trp Xaa

                325                 330                 335

Xaa Tyr Lys Xaa Gly Phe Gly Xaa Xaa Xaa Gly Glu Xaa Trp Leu Gly

            340                 345                 350

Xaa Glu Xaa Xaa Xaa Xaa Xaa Xaa Xaa Xaa Xaa Xaa Xaa Xaa Xaa Xaa

        355                 360                 365

Xaa Xaa Xaa Xaa Asp Trp Xaa Xaa Xaa Xaa Xaa Xaa Xaa Xaa Xaa Xaa

    370                 375                 380

Xaa Xaa Xaa Xaa Xaa Xaa Xaa Xaa Xaa Xaa Tyr Xaa Xaa Xaa Leu Xaa

385                 390                 395                 400

Xaa Xaa Xaa Gly Xaa Xaa Xaa Xaa Xaa Xaa Xaa Xaa Xaa Xaa Xaa Xaa

                405                 410                 415

Xaa Phe Xaa Thr Xaa Xaa Xaa Xaa Xaa Xaa Xaa Xaa Xaa Xaa Xaa Cys

            420                 425                 430

Xaa Xaa Xaa Xaa Xaa Gly Gly Trp Trp Xaa Xaa Xaa Cys Xaa Xaa Xaa

        435                 440                 445

Asn Leu Asn Gly Xaa Xaa Xaa Xaa Xaa Xaa Xaa Xaa Xaa Xaa Lys His

    450                 455                 460

"
Gln Asp Gly Ile Phe Trp Ala Glu Tyr Arg Gly Gly Ser Tyr Ser Leu
"
465                 470                 475                 480
"

"
Arg Ala Val Gln Met Met Ile Lys Pro Ile Asp
"
                485                 490
"

"

"

"

## Description

This application claims the benefit of U.S. Provisional Application No. 60/094,033, filed July 24, 1998.

This invention relates to recombinant DNA technology. In particular the invention pertains to angiogenesis-related genes, proteins, pharmaceutical compositions, and pharmaceutical and other uses thereof.

Identifying the role of specific proteins in development and differentiation of higher organisms, including humans, has become a major challenge for modern molecular biology. For example, a secreted protein termed "Scabrous" in *Drosophila,* plays an important role in defining positional aspects of developing neurogenic regions. Interestingly, sequence comparisons reveal that Scabrous is related to the angiopoietin family of mammalian proteins, which are necessary for normal angiogenesis, i.e. vascular network formation.

Angiogenesis occurs during embryonic development and continues throughout life. During embroyonic development, a primitive vascular network forms by processes that involve regulated proliferation, differentiation, migration, and association of endothelial cells. Subsequent angiogenic processes remodel this primary network to form a mature cardiovascular system.

During adulthood angiogenesis continues to be important, for example, in normal tissue repair and during the menstrual cycle. Unfortunately, the process also facilitates undesirable pathogenic effects, for example, the proliferation and metastasis of tumor cells.

Mechanistically, angiogenesis involves multiple proteins, including tyrosine kinase receptors and protein ligands that bind the receptors to stimulate or inhibit angiogenesis. *See e.g.* J. Folkman and P. D'Amore, Cell 87, 1153-1155, 1996; S. Davies et al. Cell, 87, 1161-1169, 1996, herein incorporated by reference. The first protein to be identified in this process was dubbed "angiopoietin 1" (See S. Davis et al. Cell, 87, 1161-1169, 1996). Angiopoietin 1 comprises a 70 KD glycosylated protein of 498 amino acid residues in human and mouse. There are at least two recognizable domains on this protein, one a fibrinogen-like region at the carboxyl end, and the other a coiled-coil domain at the amino terminus. Coiled-coil regions typically are involved in the assembly of a protein into multimeric structures. It is thought that the coiled-coil region of angiopoietin 1 may serve to assemble the fibrinogen-like domain into a multimeric structure. The fibrinogen-like domain of angiopoietin 1 and other related molecules is probably the receptor binding portion of this class of molecules (See Valenzuela et al. Proc. Nat. Acad. Sci. 96, 1904-1909, 1999).

Angiopoietin 1 appears to function in angiogenesis by binding the Tie2 receptor and promoting tyrosine phosphorylation thereof. Interestingly, angiopoietin 1 does not stimulate the growth of endothelial cells in vitro, as does VEGF and fibroblast growth factor, for example. Thus, angiopoietin 1 would seem to play a role other than as a mitogen. The evidence to date favors a role in regulating the assembly of non-endothelial vessel wall components, and in controlling the maturation and stabilization of blood vessels (See C. Suri et al. Cell, 87, 1171-1180, 1996).

Angiopoietin 1 has a naturally occurring antagonist, termed angiopoietin 2, which blocks the ability of angiopoietin 1 to bind its receptor, Tie 2. Angiopoietin 2 appears to be expressed only at sites of vascular remodeling in the adult. (Maisonpierre et al. Science, 277, 55-60, 1997) The other receptor known to be required for normal angiogenesis is Tiel *(See e.g.* D. Hanahan, *Science,* 277, 48, 1997). The Tie2 tyrosine kinase enables endothelial cells to recruit stromal cells for the encasement of endothelial tubes. Tiel, on the other hand, may be involved in the control of fluid exchange across capillary boundaries, and in hemodynamic stress resistance.

Recently, two additional angiopoietin-related proteins were identified in mouse and human, termed angiopoietin 3 and angiopoietin 4 (See Valenzuela et al. Proc. Nat. Acad. Sci. 96, 1904-1909, 1999). These molecules also bind the Tie2 receptor, angiopoietin 3 as an antagonist, but angiopoietin 4 as an agonist.

While the full complement of molecular players involved in angiogenesis are yet to be defined, it is probable that additional angiopoietin-like ligands and receptors exist. Accordingly, there is a need to provide other angiopoietin related molecules, as well as methods of making and using thereof. The present invention relates to an angiopoietin-like gene and its encoded protein, as well as to pharmaceutical and other uses thereof.

The present invention provides angiopoietin-related nucleic acid molecules and their encoded proteins. The genes and proteins of the invention are referred to herein as "Scarface 2." Scarface 2 is a member of a family of related Scarface proteins that are the subject of co-pending U.S. patent applications entitled "Angiopoietin related gene sequence Scarface 1," filed 1 June 1999, and "Angiopoietin related gene sequence Scarface 3," filed 1 June 1999, the entire contents of both applications herein being incorporated by reference. A specific Scarface 2 gene sequence is designated herein by SEQ ID NO:1, which originates from a human source. The present invention further provides methods for treating malignant and non-malignant tumors, and/or preventing tumor growth using Scarface 2, or related protein, or analog thereof. The invention also provides a method for treating soft tissue cancers including leukemias, and methods for suppressing the growth of stem cells including hematopoietic progenitor cells, and/or to prevent the expansion of said cells during chemotherapy or radiotherapy.

The Scarface 2 proteins are structurally and functionally related to the other members of the Scarface famile and to the angiopoietins. The Scarface proteins including Scarface 2 are useful, for example, as potential pharmaceutical compounds for modulating angiogenesis. Scarface 2 is also useful in screening for compounds that bind said proteins, and/or as an antigen to raise a specific antibody, as a tool to modulate angiogenesis, or a means for identifying Scarface 2 and related proteins in biological tissues or other biological samples.

In one embodiment, the present invention relates to a method for treating or inhibiting tumor growth, including soft tissue tumors such as leukemias by administration of a therapeutically effective amount of Scarface 2 or analog thereof.

In another embodiment the present invention relates to a method for suppressing the growth of hematopoietic stem cells prior to and/or during chemotherapy or radiotherapy.

In another embodiment, the present invention relates to a method for suppressing tumor growth or treating a mammal with a solid tumor, or treating cancer by administering to a mammal in need thereof an effective amount of Scarface 2.

In another embodiment, the present invention relates to a substantially purified Scarface 2 protein or fragment thereof.

In another embodiment, the present invention relates to a substantially purified protein having an amino acid sequence that is encoded by a nucleic acid sequence that hybridizes to SEQ ID NO:1 under high stringency conditions, said protein having Scarface 2 activity.

In another embodiment, the present invention relates to a substantially purified Scarface 2 protein from a human source wherein said protein has an amino acid sequence designated herein as SEQ ID NO:2.

In another embodiment, the present invention relates to an analog of Scarface 2, or a protein that is structurally and functionally related to Scarface 2, said analog or protein designated herein as SEQ ID NO:3.

In still another embodiment, the present invention relates to a substantially purified protein preferably having an amino acid sequence that is at least 70% identical to SEQ ID NO:2, said protein having Scarface 2 activity in vivo or in vitro.

In another embodiment, the present invention relates to chimeric or fusion proteins comprising all or part of a Scarface 2 protein or analog thereof.

In another embodiment, the present invention relates to isolated nucleic acids encoding a Scarface 2 protein.

In another embodiment, the present invention relates to isolated nucleic acids encoding a protein designated herein as SEQ ID NO:2.

In another embodiment, the present invention relates to an isolated nucleic acid that encodes a Scarface 2 protein wherein said nucleic acid is SEQ ID NO:1.

In still another embodiment the present invention relates to a vector comprising an isolated nucleic acid of the present invention, wherein said isolated nucleic acid compound may be operably-linked to a promoter sequence.

In another embodiment, the present invention relates to a host cell containing a vector of the present invention.

In yet another embodiment, the present invention relates to a pharmaceutical formulation comprising as an active ingredient a therapeutically effective amount of an Scarface 2 peptide or protein, associated with one or more pharmaceutically acceptable carriers, excipients, or diluents thereof.

In a further embodiment, the present invention relates to a pharmaceutical formulation comprising an Scarface 2 agonist or antagonist associated with one or more pharmaceutically acceptable carriers, excipients, or diluents thereof.

In another embodiment, the present invention relates to Scarface 2 or a pharmaceutically acceptable salt thereof, for use as a pharmaceutical.

In another embodiment, the present invention relates to Scarface 2, or a pharmaceutically acceptable salt thereof, for use as a pharmaceutical to treat leukemia or to inhibit tumor growth.

In still another embodiment the present invention relates to a method for identifying an antagonist of an Scarface 2 peptide, wherein said antagonist interferes in the binding of said Scarface 2 peptide to its receptor, comprising the steps of: mixing an Scarface 2 preparation with a test sample, or a control sample; monitoring the binding of said Scarface 2 peptide to its receptor, by any suitable means; and comparing the level of binding of Scarface 2 to its receptor in the test sample to the level of said binding in the control sample.

In still another embodiment the present invention relates to a method for identifying an agonist of an Scarface 2 protein or peptide, wherein said agonist promotes or substitutes for Scarface 2 in the binding of a Scarface 2 receptor, comprising the steps of: mixing an Scarface 2 preparation with a test sample, or a control sample; monitoring the binding of said Scarface 2 peptide to its receptor, by any suitable means; and comparing the level of binding of Scarface 2 to its receptor in the test sample to the level of said binding in the control sample.

In another embodiment, the present invention relates to a method for modulating angiogenesis by administration of an Scarface 2 peptide.

In still another embodiment, the present invention relates to an antibody that selectively binds to an Scarface 2 peptide or protein.

### Definitions

The term "analog" or "functional analog" refers to a related modified form of Scarface 2, in which at least one amino acid substitution, deletion, or addition has been made such that said analog retains substantially the same biological activity as the unmodified form, in vivo and/or in vitro.

The term "angiogenesis" refers generally to the formation and differentiation of blood vessels.

The term "antibody" as used herein describes antibodies, fragments of antibodies (such as, but not limited, to Fab, Fab', Fab₂', and Fv fragments), and chimeric, humanized, veneered, resurfaced, or CDR-grafted antibodies capable of binding antigens of a similar nature as the parent antibody molecule from which they are derived.

The term "chimeric protein" or "fusion protein" refers to a protein that is related to Scarface 2 comprising a hybrid protein or peptide comprising part or all of a Scarface 2 protein or analog thereof linked to a heterologous protein. The heterologous protein can be fused to the N-terminus or to the C-terminus of Scarface 2. A preferred chimeric protein comprises the fibrinogen-like domain of a Scarface 2 protein fused to a constant region of an antibody, for example, an IgG constant region. Also contemplated by the present invention are fusion proteins comprising a Scarface 2 fibrinogen-like domain fused to a coiled-coil domain from another Scarface protein, for example Scarface 1 or Scarface 3.

The term "coiled-coil domain" refers to a region of Scarface 2 at the amino terminus from about residues 1 through 276 of SEQ ID NO:2; alternatively from about residue 63 through residue 249 of SEQ ID NO:2

The terms "complementary" or "complementarity" as used herein refer to the capacity of purine and pyrimidine nucleotides to associate through hydrogen bonding to form double stranded nucleic acid molecules. The following base pairs establish complementary relationships: guanine and cytosine; adenine and thymine; and adenine and uracil. As used herein, "complementary" means that the aforementioned relationship applies to substantially all base pairs comprising two single-stranded nucleic acid molecules over the entire length of said molecules. "Partially complementary" may refer to the aforementioned relationship in which one of two single-stranded nucleic acid molecules is shorter in length than the other such that a portion of one of the molecules remains single-stranded, or to a situation in which equal length single-stranded molecules do not form complementary base pair relationships at each position.

The term "conservative substitution" or "conservative amino acid substitution" refers to a replacement of one or more amino acid residue(s) in a parent protein as stipulated by Table 1.

The term "fibrinogen-like domain" refers to a highly conserved region typically found at the carboxy end of a protein molecule, said domain being related structurally and/or by sequence to the homologous region of a fibrinogen protein molecule. For example, the fibrinogen-like domain of Scarface 2 resides between the position approximating residues 277 through 462 of SEQ ID NO:2. The fibrinogen-like domain may be important for the biological activity of Scarface 2 and related molecules.

The term "fragment thereof" refers to a fragment, piece, or sub-region of a nucleic acid or protein molecule whose sequence is disclosed herein, such that the fragment comprises 5 or more amino acids, or 10 or more nucleotides that are contiguous in the parent protein or nucleic acid molecule. "Fragment thereof" may or may not retain biological activity. A fragment of a protein disclosed herein could be used as an antigen to raise a specific antibody against the parent protein molecule, or it could retain biological activity as a fragment of the native protein molecule. With reference to a nucleic acid molecule, "fragment thereof" refers to 10 or more contiguous nucleotides, derived from a parent nucleic acid. The term also encompasses the complementary sequence. For example, if the fragment entails the sequence 5'-AGCTAG-3', then "fragment thereof" would also include the complementary sequence, 3'-TCGATC-5'.

The term "from about" as used herein contemplates one or more; alternatively from one to five, one to ten, one to twenty; or from twenty to fifty residues before or after a designated point or residue of a sequence of amino acids or nucleotides. For example, from about amino acid position 100 of SEQ ID NO:2 would contemplate the following subset of a larger set of molecules having an amino terminal end beginning alternatively at position 99, position 98, position 97, position 96, position 95, position 94, position 93, position 92, position 91, or position 90; or alternatively beginning at position 101, position 102, position 103, position 104, position 105, position 106, position 107, position 108, position 109, or position 110 of SEQ ID NO:2.

The term "functional fragment" or "functionally equivalent fragment", as used herein, refers to a region, or fragment of a full length protein, or sequence of amino acids that, for example, comprises an active site of a protein or enzyme, or a binding/recognition site, or any other amino acid sequence motif, said fragment relating to biological function. Functional fragments are capable of providing a substantially similar biological activity as a native full length protein, *in vivo* or *in vitro,* viz. the capacity to modulate angiogenesis, or the ability to promote phosphorylation of the Tie2 receptor on, for example, epithelial cells. Functional fragments can be made by biochemical means or by cloning technology, or as the natural products of alternative splicing mechanisms.

The term "structurally and functionally related" is used herein to describe peptides or proteins that are related structurally and functionally to Scarface 2. Said proteins, designated herein by SEQ ID NO:3, generally constitute modifications of SEQ ID NO:2 in which conservative or other suitable amino acid changes are introduced by natural mutation or by recombinant DNA techniques, or by other means, or by deletion of one to ten contiguous amino acid residues of SEQ ID NO:2. Generally, structurally and functionally related proteins are greater than 50% identical with SEQ ID NO:2, alternatively greater than 65% identical, preferably between 70% to 100% identical with SEQ ID NO:2; still more preferably between 85% to 95% identical; more preferably still, at least 90% identical; most preferably at least 95% identical; still most preferably between 98% to 100% identical to SEQ ID NO:2. The overall percent identity between nucleic acid molecules or protein molecules is determined by any suitable comparison algorithm, well known to the skilled artisan. The overall percent identity is determined by the number of residues that are identical between optimally aligned nucleic acids (or proteins), divided by the total length (including gaps if present) of the shortest sequence of the pair or group being compared. Percent identity can also refer to, or be expressed as, a local comparison of regions, motifs, or blocks within molecules. Local identities between molecules may be greater than 80% to 90%, while surrounding regions may be less conserved, say less than 50% identical. Such variability of conservation of sequence along the length of molecules can result in low percent identity overall, but a high percent identity within locally defined regions. Examples of locally conserved regions might include binding sites, or catalytic sites. For example, the invention is intended to include analogs of Scarface 2 in which said analogs preserve at least 40% identity with the region comprising residues from about 277 through 462 of SEQ ID NO:2.

Nucleic acid or protein sequences are optimally aligned so as to achieve the greatest degree of similarity, allowing for gaps, using any suitable algorithm, for example, a dynamic programming algorithm (See e.g. Smith and Waterman, J. Mol. Biol. 147, 195 (1985), BLASTA (Altschul et al. J. Mol. Biol. 215, 403 (1990); or FASTA (Lipman & Pearson, Science, 227, 1435 (1985), herein incorporated by reference. Such alignments are carried out with the appropriate algorithmic parameters set to maximize the alignment score obtained for a pair of sequences being compared. For example, using a BLAST algorithm, the Hsp parameter can be set at from 10 to 11 for comparing nucleic acids and at 3 when comparing amino acid sequences. Other parameters that can be varied include the expectation value parameter, most typically set to 10. The skilled artisan is aware of the values most appropriate so as to maximize the alignment between any two or more sequences. Said structurally and functionally related proteins retain the biological activity of Scarface 2, such as the ability to modulate angiogenesis, or the ability to stimulate phosphorylation of Tie2, or to inhibit solid tumor growth in vivo, or the ability to suppress the growth of hematopoietic progenitor cells (*See e.g.* D. Dumont et al. *Oncogene* 7, 1471-1480, 1992).

The term "host cell" refers to any eucaryotic or procaryotic cell that is suitable for propagating and/or expressing a cloned gene contained on a vector that is introduced into said host cell by, for example, transformation or transfection, or the like.

Specific examples of Scarface 2 genes and/or proteins are referred to herein as "Scarface-2" (SEQ ID NO:1/SEQ ID NO:2). Scarface 2 molecules are related to angiopoietin 1, which mediates angiogenesis during embryogenic and later development.

The term "homolog" or "homologous" describes the relationship between different nucleic acid molecules, or amino acid sequences, in which said sequences or molecules are related by partial identity or chemical/physical similarity at one or more blocks or regions within said molecules or sequences.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of homology, the stringency of hybridization, and the length of hybridizing strands.

The term "isolated nucleic acid compound" refers to any RNA or DNA sequence, however constructed or synthesized, which is locationally distinct from its natural location and which comprises a subgenomic and/or subchromosomal fragment

The symbol "N" in a nucleic acid sequence refers to adenine ("A"), guanine ("G"), cytosine ("C"), thymine (T"), or uracil ("U").

The term "modulate" or "modulating" refers to stimulating or inhibiting the object thereof, for example, stimulating or inhibiting angiogenesis.

The term "nucleic acid probe" or "probe" as used herein is a labeled nucleic acid compound which hybridizes with another nucleic acid compound. "Nucleic acid probe" means a single stranded nucleic acid sequence that will combine with a complementary or partially complementary single stranded target nucleic acid sequence to form a double-stranded molecule. A nucleic acid probe may be an oligonucleotide or a nucleotide polymer. A probe will usually contain a detectable moiety which may be attached to the end(s) of the probe or be internal to the sequence of the probe.

The term "orthologue" or "orthologous" refers to two or more genes or proteins from different organisms that exhibit sequence homology.

The term "paralogue" or "paralogous" refers to two or more genes or proteins within a single organism that exhibit sequence homology.

The term "plasmid" refers to an extrachromosomal genetic element. The plasmids disclosed herein are commercially available, publicly available on an unrestricted basis, or can be constructed from readily available plasmids in accordance with published procedures.

A "primer" is a nucleic acid fragment which functions as an initiating substrate for enzymatic or synthetic elongation of, for example, a nucleic acid molecule.

The term "promoter" refers to a nucleic acid sequence that directs transcription, for example, of DNA to RNA. An inducible promoter is one that is regulatable by environmental signals, such as carbon source, heat, or metal ions, for example. A constitutive promoter generally operates at a constant level and is not regulatable.

The terms "protein" and "peptide" are used interchangeably herein with reference to a biopolymer comprising a plurality of covalently linked amino acid residues.

The term "recombinant DNA cloning vector" as used herein refers to any autonomously replicating agent, including, but not limited to, plasmids and phages, comprising a DNA molecule to which one or more additional DNA segments can or have been incorporated.

The term "recombinant DNA expression vector" or "expression vector" as used herein refers to any recombinant DNA cloning vector, for example a plasmid or phage, in which a promoter and other regulatory elements are present thereby enabling transcription of an inserted DNA, which may encode a protein.

The term "Scarface 2 activity" or "Scarface 2-like activity" refers to one or more biological activities exhibited by Scarface 2, for example, the ability to inhibit tumor growth in vivo, or tumor cell proliferation in vitro, or the ability to stimulate phosphorylation of Tie 2, or exhibiting a myelosuppressive effect in vivo or in vitro, or any other effect of Scarface 2.

The term "stem cell" refers generally to an unspecialized cell type that gives rise to differentiated cells. For example, hematopoietic stem cells in bone marrow give rise to cells of the blood, e.g. B-cells and red blood cells.

The term "stringency" refers to hybridization conditions. High stringency conditions disfavor non-homologous base pairing. Low stringency conditions have the opposite effect. Stringency may be altered, for example, by temperature and salt concentration.

The term "low stringency" refers to conditions that comprise, for example, a temperature of about 37° C or less, a formamide concentration of less than about 50%, and a moderate to low salt (SSC) concentration; or, alternatively, a temperature of about 50° C or less, and a moderate to high salt (SSPE) concentration, for example 1M NaCl.

The term "high stringency" refers to conditions that comprise, for example, a temperature of about 42° C or less, a formamide concentration of less than about 20%, and a low salt (SSC) concentration; or, alternatively, a temperature of about 65° C, or less, and a low salt (SSPE) concentration. For example, high stringency conditions comprise hybridization in 0.5 M NaHPO₄, 7% sodium dodecyl sulfate (SDS), 1 mM EDTA at 65°C (Ausubel, F.M. *et al.* Current Protocols in Molecular Biology, Vol. I, 1989; Green Inc. New York, at 2.10.3).

The term "SSC" comprises a hybridization and wash solution. A stock 20X SSC solution contains 3M sodium chloride, 0.3M sodium citrate, pH 7.0.

The term "SSPE" comprises a hybridization and wash solution. A 1X SSPE solution contains 180 mM NaCl, 9mM Na₂HPO₄, 0.9 mM NaH₂PO₄ and 1 mM EDTA, pH 7.4.

The term "substantially pure," used in reference to a peptide or protein, means substantial separation from other cellular and non-cellular molecules, including other protein molecules. A substantially pure preparation would be about at least 85% pure; preferably about at least 95% pure. A "substantially pure" protein can be prepared by a variety of techniques, well known to the skilled artisan, including, for example, the IMAC protein purification method.

The term "vector" as used herein refers to a nucleic acid compound used for introducing exogenous or endogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

The various restriction enzymes disclosed and described herein are commercially available and the manner of use of said enzymes including reaction conditions, cofactors, and other requirements for activity are well known to one of ordinary skill in the art. Reaction conditions for particular enzymes were carried out according to the manufacturer's recommendation.

The Scarface 2 proteins are related by sequence to mammalian angiopoietin 1 and angiopoietin 2, and to the *Drosophila* "Scabrous" protein. Scarface 2 is expressed in multiple tissues and cell types including kidney tubule cells and liver. Those skilled in the art will recognize that owing to the degeneracy of the genetic code, numerous "silent" substitutions of nucleotide base pairs could be introduced into the sequence(s) identified herein without altering the identity of the encoded amino acid(s) or protein or peptide product. All such substitutions are intended to be within the scope of the invention.

Specific examples of a Scarface 2 cDNA and protein are identified herein as SEQ ID NO:1 and SEQ ID NO:2, respectively. Scarface 2 (SEQ ID NO:2) comprises multiple functional domains including a fibrinogen-like domain from approximately about residue 277 through 462 of SEQ ID NO:2, and a coiled-coil domain at the amino terminal end from approximately residues 1 through 269; alternatively from about residue 63 through residue 249 of SEQ ID NO:2.

Also contemplated by the present invention are analogs of Scarface 2, or proteins and/or peptides that are structurally and functionally related to Scarface 2, identified herein as SEQ ID NO:3. For example, proteins and peptides that are related to SEQ ID NO:2 comprise conservative amino acid substitutions, replacements, deletions, or insertions, at one or more amino acid positions within SEQ ID NO:2, generally in accordance with Table 1. Modifications of Scarface 2 made in accordance with Table 1 are generally expected to retain the biological activity of SEQ ID NO:2 based on the generally recognized substitutability of certain amino acids (*See e.g.* M. Dayhoff, In Atlas of Protein Sequence and Structure, Vol. 5, Supp. 3, pgs 345-352, 1978). Functionality can be tested in an assay that measures phosphorylation of the Tie2 receptor, or the ability to modulate angiogenesis, or to inhibit tumor growth, for example.

| ORIGINAL RESIDUE | EXEMPLARY SUBSTITUTIONS |
|---|---|
| ALA | SER, THR |
| ARG | LYS |
| ASN | HIS, SER |
| ASP | GLU, ASN |
| CYS | SER |
| GLN | ASN, HIS |
| GLU | ASP, GLU |
| GLY | ALA, SER |
| HIS | ASN, GLN |
| ILE | LEU, VAL, THR |
| LEU | ILE, VAL |
| LYS | ARG, GLN, GLU, THR |
| MET | LEU, ILE, VAL |
| PHE | LEU, TYR |
| SER | THR, ALA, ASN |
| THR | SER, ALA |
| TRP | ARG, SER |
| TYR | PHE |
| VAL | ILE, LEU, ALA |
| PRO | ALA |

Analogs of Scarface 2, designated herein by SEQ ID NO:3, comprise single or multiple amino acid substitutions in the region between amino acid residues 277 through 463 of SEQ ID NO:2, wherein:
the residue at position 277 is alternatively Pro, Phe, or Trp;
the residue at position 278 is alternatively Ala, Lys, or Arg;
the residue at position 279 is alternatively Glu or Asp;
the residue at position 281 is alternatively Thr, Ala, Gln, or Leu;
the residue at position 282 is alternatively Thr, Asp, Glu, or Gln;
the residue at position 283 is alternatively Ile, Leu, Ala, or Val;
the residue at position 284 is alternatively Tyr, Phe, Leu, or Lys;
the residue at position 285 is alternatively Asn, Gln, Lys, or Glu;
the residue at position 286 is alternatively Arg, Ala, Val, Ser, or Asp;
the residue at position 288 is alternatively Glu, His, or Phe;
the residue at position 289 is alternatively His, Arg, Ser, Asn, Thr, or Asp;
the residue at position 290 is alternatively Thr, Lys, Val or Gln;
the residue at position 291 is alternatively Ser, or Asn;
the residue at position 292 is alternatively Gly or Ser;
the residue at position 293 is alternatively Met, Ile, or Leu;
the residue at position 294 is alternatively Tyr, or Phe;
the residue at position 295 is alternatively Ala, Thr, Leu, Met or Glu;
the residue at position 296 is alternatively Ile, Val or Leu;
the residue at position 297 is alternatively Arg, Lys, Thr, Tyr, or Gln;
the residue at position 298 is alternatively Pro, Phe, or Ile;
the residue at position 299 is alternatively Ser, Gln, Glu or Asn;
the residue at position 300 is alternatively Asn, or Gly;
the residue at position 301 is alternatively Ser, Thr, or Met;
the residue at position 302 is alternatively Asn, Thr, Pro;
the residue at position 303 is alternatively Gln, Glu, Pro, Arg, or Gly;
the residue at position 304 is alternatively Val, Pro, Leu, or Glu;
the residue at position 305 is alternatively Phe, Met, Ile, or Lys;
the residue at position 306 is alternatively His, Lys, Gln, or Leu;
the residue at position 307 is alternatively Val, Leu, or Ala;
the residue at position 308 is alternatively Tyr, Phe, Trp, or Asn;
the residue at position 310 is alternatively Asp, Asn, Glu or Lys;
the residue at position 311 is alternatively Val, Met, Asn, or Gln;
the residue at position 312 is alternatively Ile, Thr, Ser, Arg, Glu, or Asp;
the residue at position 313 is alternatively Ser, Leu, His, Ala, or Val;
the residue at position 314 is alternatively Asp, Gly, or Asn;
the residue at position 315 is alternatively Gly, Pro, or Asp;
the residue at position 316 is alternatively Ser, or Gly;
the residue at position 317 is alternatively Pro, or Gly;
the residue at position 320 is alternatively Leu, Val or Ile;
the residue at position 323 is alternatively His, Arg, or Lys;
the residue at position 325 is alternatively Ile, Glu, Leu, Thr, or His;
the residue at position 329 is alternatively Gln, Val, or Leu;
the residue at position 330 is alternatively Asn, or Asp;
the residue at position 332 is alternatively Asn, Phe, or Gln;
the residue at position 333 is alternatively Glu, or Arg;
the residue at position 334 is alternatively Thr, Gly, Asn, or Pro;
the residue at position 336 is alternatively Glu, or Lys;
the residue at position 337 is alternatively Asn, Glu, Thr, or Ala;
the residue at position 340 is alternatively Tyr, Met, Val, Gln, Lys, or Ala;
the residue at position 344 is alternatively Arg, Asn, or Asp;
the residue at position 345 is alternatively Leu, Pro, or Ile;
the residue at position 346 is alternatively Asp, Ser, or His;
the residue at position 349 is alternatively Phe, or Tyr;
the residue at position 353 is alternatively Leu, or Asn;
the residue at position 355 is alternatively Lys, Asn, or Phe;
the residue at position 356 is alternatively Ile, or Val;
the residue at position 357 is alternatively Tyr, His, Ser, or Phe;
the residue at position 358 is alternatively Ser, Met, Trp, Gln, or Ala;
the residue at position 359 is alternatively Ile, or Leu;
the residue at position 360 is alternatively Val, Thr, Ser, or Met;
the residue at position 361 is alternatively Lys, Gly, Asn, or Ser;
the residue at position 362 is alternatively Gln, Asp;
the residue at position 363 is alternatively Ser, Arg, Asp, Gly, or Gln;
the residue at position 364 is alternatively Asn, Arg, or Gln;
the residue at position 365 is alternatively Tyr, or Ser;
the residue at position 366 is alternatively Val, Met, Lys, or Arg;
the residue at position 368 is alternatively Arg, Leu, Ala, or Lys;
the residue at position 369 is alternatively Ile, or Val;
the residue at position 370 is alternatively Glu, His, Gln, or Thr;
the residue at position 371 is alternatively Leu, or Met;
the residue at position 372 is alternatively Glu, Arg, Lys, or Met;
the residue at position 375 is alternatively Lys, Ser, Glu, or Asp;
the residue at position 376 is alternatively Asp, or Gly;
the residue at position 377 is alternatively Asn, Lys, or Arg;
the residue at position 378 is alternatively Lys, Ala, Glu, or Arg;
the residue at position 379 is alternatively His, Glu, Val, or Ala;
the residue at position 380 is alternatively Tyr, Phe, or Leu;
the residue at position 381 is alternatively Ile, Leu, Ala, or Ser;
the residue at position 382 is alternatively Glu, Leu, or Gln;
the residue at position 383 is alternatively Tyr, or Phe;
the residue at position 384 is alternatively Ser, Ala, Asp, or Glu;
the residue at position 385 is alternatively Ser, His, or Arg;
the residue at position 386 is alternatively Phe, or Val;
the residue at position 387 is alternatively Tyr, His, or Arg;
the residue at position 388 is alternatively Leu, or Ile;
the residue at position 389 is alternatively Gly, Glu, or Ser;
the residue at position 390 is alternatively Asn, Pro, Ser, or Gly;
the residue at position 391 is alternatively His, or Glu;
the residue at position 392 is alternatively Glu, Asp, Ser, or Lys;
the residue at position 393 is alternatively Thr, Gln, Leu, or Glu;
the residue at position 394 is alternatively Asn, Tyr, Phe, or Ala;
the residue at position 396 is alternatively Thr, Ser, Arg, or Lys;
the residue at position 397 is alternatively Leu, or Ile;
the residue at position 398 is alternatively His, Arg, Gln, or Tyr;
the residue at position 400 is alternatively Val, Ala, Gly,or Lys;
the residue at position 401 is alternatively Ala, Gly, Arg, Thr, or Pro;
the residue at position 402 is alternatively Ile, Val, Tyr, Leu, or His;
the residue at position 403 is alternatively Thr, Ala, Gln, or His;
the residue at position 405 is alternatively Asn, Thr, or Gln;
the residue at position 406 is alternatively Val, Ala, or Leu;
the residue at position 407 is alternatively Pro, or Gly;
the residue at position 408 is alternatively Asn, Asp, Ser, or Thr;
the residue at position 409 is alternatively Ala, Ser, or Val;
the residue at position 410 is alternatively Ile, Leu, Phe, Met or Pro;
the residue at position 411 is alternatively Pro, Ile, Ser, Thr, or Met;
the residue at position 412 is alternatively Glu, Trp, Ser, Leu, or Gln;
the residue at position 413 is alternatively Asn, His, Pro, or Gly;
the residue at position 414 is alternatively Lys, Asn, or Leu;
the residue at position 415 is alternatively Asp, Gly, or Ser;
the residue at position 416 is alternatively Leu, Asn, Lys, Ala, or Val;
the residue at position 417 is alternatively Val, Gln, Asp, or Pro;
the residue at position 419 is alternatively Ser, or Thr;
the residue at position 421 is alternatively Trp, Lys, or Leu;
the residue at position 422 is alternatively His, or Asp;
the residue at position 423 is alternatively Arg, Gly, Ala, or Gln;
the residue at position 424 is alternatively His, or Asp;
the residue at position 425 is alternatively Lys, Asn, or His;
the residue at position 426 is alternatively Ala, or Asp;
the residue at position 427 is alternatively Lys, Asn, Val, Met, or Leu;
the residue at position 428 is alternatively Gly, Arg, Tyr, or Cys;
the residue at position 429 is alternatively His, Met, Ile, Thr, Ala, or Asp;
the residue at position 430 is alternatively Phe, Cys, Gly, or Lys;
the residue at position 431 is alternatively Asn, or Lys;
the residue at position 433 is alternatively Pro, Ala, or Ser;
the residue at position 434 is alternatively Glu, His, Leu, Gln, or Lys;
the residue at position 435 is alternatively Gly, Ser, Phe, Tyr, or Met;
the residue at position 436 is alternatively Tyr, Leu, Gln, or His;
the residue at position 437 is alternatively Ser, Lys, or Thr;
the residue at position 442 is alternatively His, Phe, or Tyr;
the residue at position 443 is alternatively Asp, Asn, or Gly;
the residue at position 444 is alternatively Glu, Ala, or Thr;
the residue at position 446 is alternatively Gly, Ala, or Ser;
the residue at position 447 is alternatively Glu, His, or pro;
the residue at position 448 is alternatively Asn, or Ser;
the residue at position 453 is alternatively Lys, Gln, Met or Val;
the residue at position 454 is alternatively Tyr, Trp, or Phe;
the residue at position 455 is alternatively Asn, Phe, or Tyr;
the residue at position 456 is alternatively Lys, Arg, Pro, or Thr;
the residue at position 457 is alternatively Pro, Ser, Gly, Gln, or Ala;
the residue at position 458 is alternatively Arg, Ile, Gly, or Arg;
the residue at position 459 is alternatively Ala, Gln, or His;
the residue at position 460 is alternatively Lys, Tyr, Asn, or Gln;
the residue at position 461 is alternatively Ser, Arg, Thr, or His;
the residue at position 462 is alternatively Lys, Gln, Ser, Asn, or Gly;

### Fragments of Scarface 2 proteins

Another embodiment of the invention provides fragments of Scarface 2 and related peptides and analogs that may or may not be biologically active. Such fragments are useful, for example, as an antigen for producing an antibody to said proteins. In some instances the fragments may be produced naturally by an autocatalytic process or by non-autocatalytic means, for example chemical or recombinant synthesis. For example, one naturally occurring fragment of Scarface 2 comprises the fibrinogen-like domain beginning from about residue 248 of SEQ ID NO:2. This fragment may be generated in vivo or in vitro by the action of serine proteases such as thrombin or trypsin, for example.

Fragments of the proteins disclosed herein may be generated by any number of suitable techniques, including chemical synthesis of any portion of SEQ ID NO:2, proteolytic digestion of said peptides, or most preferably, by recombinant DNA mutagenesis techniques, well known to the skilled artisan. *See. e.g.* K. Struhl, "Reverse biochemistry: Methods and applications for synthesizing yeast proteins *in vitro," Meth. Enzymol.* 194, 520-535. For example, in a preferred method, a nested set of deletion mutations are introduced into a gene or gene fragment (e.g. SEQ ID NO:1) encoding an Scarface 2 peptide such that varying amounts of the peptide coding region are deleted, either from the amino terminal end, or from the carboxyl end of the protein molecule. This method can also be used to create internal fragments of the intact protein in which both the carboxyl and amino terminal ends are removed. Several appropriate nucleases can be used to create such deletions, for example *Bal*31, or in the case of a single stranded nucleic acid molecule, mung bean nuclease. For simplicity, it is preferred that the Scarface 2 gene be cloned into a single-stranded cloning vector, such as bacteriophage M13, or equivalent. If desired, the resulting deletion fragments can be subcloned into any suitable vector for propagation and expression in any suitable host cell.

Functional fragments of the proteins of this invention may be produced as described above, preferably using cloning techniques to engineer smaller versions of the intact gene, lacking sequence from the 5' end, the 3' end, from both ends, or from an internal site. Smaller fragments of the genes of this invention may be used to produce the encoded peptides, which may be tested for biological activity using any suitable assay, for example, the ability of a protein fragment to stimulate phosphorylation of Tie2.

### Gene Isolation Procedures

Those skilled in the art will recognize that the Scarface 2 genes could be obtained by a plurality of recombinant DNA techniques including, for example, hybridization, polymerase chain reaction (PCR) amplification, or *de novo* DNA synthesis. (See e.g., T. Maniatis *et al.* Molecular Cloning: A Laboratory Manual, 2d Ed. Chap. 18 (1989)).

Methods for constructing cDNA libraries in a suitable vector such as a plasmid or phage for propagation in procaryotic or eucaryotic cells are well known to those skilled in the art. [*See e.g.* Maniatis *et al. Supra*]. Suitable cloning vectors are well known and are widely available.

The Scarface 2 genes, or fragments thereof, can be isolated from any tissue in which said genes are expressed. PCR analysis and Northern blot analysis revealed that Scarface-2 is expressed in multiple tissues including, for example, brain, heart, kidney, liver, lung, pancrease, placenta, colon, ovary, leukocyte, prostate, muscle, bone marrow, spleen, lymph node, thymus, and fetal liver (data not shown).

In one method for Scarface 2 gene isolation, total mRNA is isolated from a suitable tissue, and first strand cDNA synthesis is carried out. Second round DNA synthesis can be carried out for the production of the second strand. If desired, the double-stranded cDNA can be cloned into any suitable vector, for example, a plasmid, thereby forming a cDNA library. Oligonucleotide primers targeted to any suitable region of the sequences disclosed herein can be used for PCR amplification of Scarface 2 genes. *See e.g.* PCR Protocols: A Guide to Method and Application, Ed. M. Innis et al., Academic Press (1990). The PCR amplification comprises template DNA, suitable enzymes, primers, and buffers, and is conveniently carried out in a DNA Thermal Cycler (Perkin Elmer Cetus, Norwalk, CT). A positive result is determined by detecting an appropriately-sized DNA fragment following agarose gel electrophoresis.

### Protein Production Methods

Another embodiment of the present invention relates to substantially purified Scarface 2 proteins or analogs, for example the Scarface 2 identified herein as SEQ ID NO:2, and including analogs thereof (SEQ ID NO:3) and fragments thereof.

Skilled artisans will recognize that the proteins of the present invention can be synthesized by a number of different methods, such as chemical methods well known in the art, including solid phase peptide synthesis or recombinant methods. Both methods are described in U.S. Patent 4,617,189, incorporated herein by reference.

The principles of solid phase chemical synthesis of polypeptides are well known in the art and may be found in general texts in the area. *See, e.g.,* H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems.

The proteins of the present invention can also be produced by recombinant DNA methods using a cloned Scarface 2 gene as template. Recombinant methods are preferred if a high yield is desired. Expression of an Scarface 2 gene can be carried out in a variety of suitable host cells, well known to those skilled in the art. For example, SEQ ID NO:1 is introduced into a host cell by any suitable means, well known to those skilled in the art. While chromosomal integration of the cloned gene is within the scope of the present invention, it is preferred that the gene be cloned into a suitable extra-chromosomally maintained expression vector so that the coding region of the Scarface 2 gene is operably-linked to a constitutive or inducible promoter.

The basic steps in the recombinant production of an Scarface 2 protein are:
a) constructing a natural, synthetic or semi-synthetic DNA encoding an Scarface 2 protein;
b) integrating said DNA into an expression vector in a manner suitable for expressing the Scarface 2 protein, either alone or as a fusion protein;
c) transforming or otherwise introducing said vector into an appropriate eucaryotic or prokaryotic host cell forming a recombinant host cell,
d) culturing said recombinant host cell in a manner to express the Scarface 2 protein; and
e) recovering and substantially purifying the Scarface 2 protein by any suitable means, well known to those skilled in the art.

### Expressing Recombinant Scarface 2 Protein in Procaryotic and Eucaryotic Host Cells

Procaryotes may be employed in the production of recombinant Scarface 2 protein. For example, the *Escherichia coli* K12 strain 294 (ATCC No. 31846) is particularly useful for the prokaryotic expression of foreign proteins. Other strains of *E. coli,* bacilli such as *Bacillus subtilis,* enterobacteriaceae such as *Salmonella typhimurium* or *Serratia marcescens,* various *Pseudomonas* species and other bacteria, such as *Streptomyces,* may also be employed as host cells in the cloning and expression of the recombinant proteins of this invention.

Promoter sequences suitable for driving the expression of genes in procaryotes include β-lactamase [*e.g.* vector pGX2907, ATCC 39344, which contains a replicon and β-lactamase gene], lactose systems [Chang et al., *Nature* (London), 275:615 (1978); Goeddel et al., *Nature* (London), 281:544 (1979)], alkaline phosphatase, and the tryptophan (trp) promoter system [vector pATHl (ATCC 37695)], which is designed to facilitate expression of an open reading frame as a trpE fusion protein under the control of the trp promoter. Hybrid promoters such as the tac promoter (isolatable from plasmid pDR540, ATCC-37282) are also suitable. Still other bacterial promoters, whose nucleotide sequences are generally known, may be ligated to DNA encoding the protein of the instant invention, using linkers or adapters to supply any required restriction sites. Promoters for use in bacterial systems also will contain a Shine-Dalgarno sequence operably-linked to the DNA encoding the desired polypeptides. These examples are illustrative rather than limiting.

The proteins of this invention may be synthesized either by direct expression or as a fusion protein comprising the protein of interest as a translational fusion with another protein or peptide which may be removable by enzymatic or chemical cleavage. It is often observed in the production of certain peptides in recombinant systems that expression as a fusion protein prolongs the lifespan, increases the yield of the desired peptide, or provides a convenient means of purifying the protein. This is particularly relevant when expressing mammalian proteins in procaryotic hosts. A variety of peptidases (e.g. enterokinase and thrombin) which cleave a polypeptide at specific sites or digest the peptides from the amino or carboxy termini (*e.g.* diaminopeptidase) of the peptide chain are known. Furthermore, particular chemicals (*e.g.* cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.,* P. Carter, "Site Specific Proteolysis of Fusion Proteins", Chapter 13, in Protein Purification: From Molecular Mechanisms to Large Scale Processes, American Chemical Society, Washington, D.C. (1990).

In addition to procaryotes, a variety of amphibian expression systems such as frog oocytes, and mammalian cell systems can be used. The choice of a particular host cell depends to some extent on the particular expression vector used. Exemplary mammalian host cells suitable for use in the present invention include HepG-2 (ATCC HB 8065), CV-1 (ATCC CCL 70), LC-MK₂ (ATCC CCL 7.1), 3T3 (ATCC CCL 92), CHO-K1 (ATCC CCL 61), HeLa (ATCC CCL 2), RPMI8226 (ATCC CCL 155), H4IIEC3 (ATCC CCL 1600), C127I (ATCC CCL 1616), HS-Sultan (ATCC CCL 1884), and BHK-21 (ATCC CCL 10), for example.

A wide variety of vectors are suitable for transforming mammalian host cells. For example, the pSV2-type vectors comprise segments of the simian virus 40 (SV40) genome required for transcription and polyadenylation. A large number of plasmid pSV2-type vectors have been constructed, such as pSV2-gpt, pSV2-neo, pSV2-dhfr, pSV2-hyg, and pSV2- -globin, in which the SV40 promoter drives transcription of an inserted gene. These vectors are widely available from sources such as the American Type Culture Collection (ATCC), 12301 Parklawn Drive, Rockville, Maryland, 20852, or the Northern Regional Research Laboratory (NRRL), 1815 N. University Street, Peoria, Illinois, 61604.

Promoters suitable for expression in mammalian cells include the SV40 late promoter, promoters from eukaryotic genes, such as, for example, the estrogen-inducible chicken ovalbumin gene, the interferon genes, the glucocorticoid-inducible tyrosine aminotransferase gene, the thymidine kinase gene promoter, and the promoters of the major early and late adenovirus genes.

Plasmid pRSVcat (ATCC 37152) comprises portions of a long terminal repeat of the Rous Sarcoma virus, a virus known to infect chickens and other host cells. This long terminal repeat contains a promoter which is suitable for use in the vectors of this invention. H. Gorman *et al., Proc. Nat. Acad. Sci. (USA),* 79, 6777 (1982). The plasmid pMSVi (NRRL B-15929) comprises the long terminal repeats of the Murine Sarcoma virus, a virus known to infect mouse and other host cells. The mouse metallothionein promoter has also been well characterized for use in eukaryotic host cells and is suitable for use in the present invention. This promoter is present in the plasmid pdBPV-MMTneo (ATCC 37224) which can serve as the starting material for the construction of other plasmids of the present invention.

Transfection of mammalian cells with vectors can be performed by a plurality of well known processes including, but not limited to, protoplast fusion, calcium phosphate co-precipitation, electroporation and the like. *See, e.g.,* Maniatis *et al., supra.*

Some viruses also make appropriate vectors. Examples include the adenoviruses, the adeno-associated viruses, the vaccinia virus, the herpes viruses, the baculoviruses, and the rous sarcoma virus, as described in U.S. Patent 4,775,624, incorporated herein by reference.

Eucaryotic microorganisms such as yeast and other fungi are also suitable host cells. The yeast *Saccharomyces cerevisiae* is the preferred eucaryotic microorganism. Other yeasts such as *Kluyveromyces lactis* and *Pichia pastoris* are also suitable. For expression in *Saccharomyces,* the plasmid YRp7 (ATCC-40053), for example, may be used. *See, e.g.,* L. Stinchcomb *et al., Nature,* 282, 39 (1979); J. Kingsman *et al., Gene,* 7, 181 (1979); S. Tschemper *et al.,* Gene, 10, 157 (1980). Plasmid YRp7 contains the TRP1 gene which provides a selectable marker for use in a trpl auxotrophic mutant.

### Purification of Recombinantly-Produced Scarface 2 Proteins

An expression vector carrying a cloned Scarface 2 gene or fragment thereof (e.g. SEQ ID NO:1) is transformed or transfected into a suitable host cell using standard methods. Cells that contain the vector are propagated under conditions suitable for expression of a recombinant Scarface 2 protein. For Example, if the recombinant gene or fragment thereof has been placed under the control of an inducible promoter, suitable growth conditions would incorporate the appropriate inducer. The recombinantly-produced protein may be purified from cellular extracts of transformed cells by any suitable means.

In a preferred process for protein purification, an Scarface 2 gene or fragment thereof is modified at the 5' end to incorporate several histidine codons. This modification produces an "histidine tag" at the amino terminus of the encoded protein, that enables a single-step protein purification method [i.e. "immobilized metal ion affinity chromatography" (IMAC)], essentially as described in U.S. Patent 4,569,794, which hereby is incorporated by reference. The IMAC method enables rapid isolation of substantially pure recombinant Scarface 2 protein starting from a crude extract of cells that express a modified recombinant protein, as described above.

### Production of Antibodies

The proteins of this invention and fragments thereof may be used in the production of antibodies. The instant invention also encompasses single-chain polypeptide binding molecules.

The production of antibodies, both monoclonal and polyclonal, in animals, especially mice, is well known in the art. *See, e.g.,* C. Milstein, Handbook of Experimental Immunology, (Blackwell Scientific Pub., 1986); J. Goding, Monoclonal Antibodies: Principles and Practice, (Academic Press, 1983). For the production of monoclonal antibodies the basic process begins with injecting a mouse, or other suitable animal, with an immunogen. The mouse is subsequently sacrificed and cells taken from its spleen are fused with myeloma cells, resulting in a hybridoma that reproduces *in vitro.* The population of hybridomas is screened to isolate individual clones, each of which secretes a single antibody species, specific for the immunogen. Each antibody obtained in this way is the clonal product of a single B cell.

Chimeric antibodies are described in U.S. Patent No. 4,816,567, the entire contents of which is herein incorporated by reference. This reference discloses methods and vectors for the preparation of chimeric antibodies. An alternative approach is provided in U.S. Patent No. 4,816,397, the entire contents of which is herein incorporated by reference. This patent teaches co-expression of the heavy and light chains of an antibody in the same host cell.

The approach of U.S. Patent 4,816,397 has been further refined in European Patent Publication No. 0 239 400. The teachings of this European patent publication are a preferred format for genetic engineering of monoclonal antibodies. In this technology the complementarity determining regions (CDRs) of a human antibody are replaced with the CDRs of a murine monoclonal antibody, thereby converting the specificity of the human antibody to the specificity of a murine antibody.

Single chain antibodies and libraries thereof are yet another variety of genetically engineered antibody technology that is well known in the art. (*See, e.g.* R.E. Bird, *et al., Science* 242:423-426 (1988); PCT Publication Nos. WO 88/01649, WO 90/18430, and WO 91/10737. Single chain antibody technology involves covalently joining the binding regions of heavy and light chains to generate a single polypeptide chain. The binding specificity of the intact antibody molecule is thereby reproduced on a single polypeptide chain.

The antibodies contemplated by this invention are useful in diagnostics, therapeutics or in diagnostic/therapeutic combinations.

The proteins of this invention or suitable fragments thereof can be used to generate polyclonal or monoclonal antibodies, and various inter-species hybrids, or humanized antibodies, or antibody fragments, or single-chain antibodies. The techniques for producing antibodies are well known to skilled artisans. (*See e.g.* A.M. Campbell, Monoclonal Antibody Technology: Laboratory Techniques in Biochemsitry and Molecular Biology, Elsevier Science Publishers, Amsterdam (1984); Kohler and Milstein, Nature 256, 495-497 (1975); Monoclonal Antibodies: Principles & Applications Ed. J.R.Birch & E.S. Lennox, Wiley-Liss, 1995.

A protein used as an immunogen may be modified or administered in an adjuvant, by subcutaneous or intraperitoneal injection into, for example, a mouse or a rabbit. For the production of monoclonal antibodies, spleen cells from immunized animals are removed, fused with myeloma cells, such as SP2/0-Ag18 cells, and allowed to become monoclonal antibody producing hybridoma cells in the manner known to the skilled artisan. Hybridomas that secrete a desired antibody molecule can be screened by a variety of well known methods, for example ELISA assay, western blot analysis, or radioimmunoassay (Lutz *et al. Exp. Cell Res.* 175, 109-124 (1988); Monoclonal Antibodies: Principles & Applications Ed. J.R.Birch & E.S. Lennox, Wiley-Liss, 1995).

For some applications labeled antibodies are desirable. Procedures for labeling antibody molecules are widely known, including for example, the use of radioisotopes, affinity labels, such as biotin or avidin, enzymatic labels, for example horseradish peroxidase, and fluorescent labels, such as FITC or rhodamine (*See e.g.* Enzyme-Mediated Immunoassay, Ed. T. Ngo, H. Lenhoff, Plenum Press 1985; Principles of Immunology and Immunodiagnostics, R.M. Aloisi, Lea & Febiger, 1988).

Labeled antibodies are useful for a variety of diagnostic applications. In one embodiment the present invention relates to the use of labeled antibodies to detect the presence of Scarface 2 proteins. Alternatively, the antibodies could be used in a screen to identify potential modulators of Scarface-2. For example, in a competitive displacement assay, the antibody or compound to be tested is labeled by any suitable method. Competitive displacement of an antibody from an antibody-antigen complex by a test compound such that a test compound-antigen complex is formed provides a method for identifying compounds that bind Scarface 2.

Other embodiments of the present invention comprise isolated nucleic acid sequences that encode the proteins of the invention, for example, SEQ ID NO:2 or SEQ ID NO:3. Also contemplated are related nucleic acids that hybridize to a Scarface 2 gene or cDNA under high stringency conditions. For example, the invention includes nucleic acids that hybridize to SEQ ID NO:1 under high stringency conditions. Such sequences may or may not encode functional proteins. In a preferred embodiment, nucleic acids that hybridize to SEQ ID NO:1 under high stringency conditions encode a protein that has similar biological function as Scarface 2, for example the ability to modulate angiogenesis, or the ability to stimulate the phosphorylation of Tie2, or the ability to suppress tumor growth. Such Scarface 2-related sequences may come, for example, from paralogous or orthologous genes.

Hybridization under high stringency conditions can be detected using any suitable method, for example, a filter-based assay such as a Southern blot analysis or a Northern blot analysis, according to well known methods. Specific hybridization means that a signal is detected on a autoradiograph, for example, that is significantly greater than background hybridization and/or to non-related sequences, for example that overall are less than 50% identical with a Scarface 2 probe. Alternatively, hybridization may be detected using a solution-based assay in which the probe and target sequences are in solution during the hybridization reaction. Hybridization means that at least 25% to 50% of the probe molecule remains bound to a target sequence after hybridization, preferably at least 50% remains bound to the target.

The Scarface 2 genes of the invention (e.g. SEQ ID NO:1) and related nucleic acid molecules may be produced by chemical synthetic methods. The synthesis of nucleic acids is well known in the art. *See, e.g.,* E.L. Brown, R. Belagaje, M.J. Ryan, and H.G. Khorana, *Methods in Enzymology,* 68:109-151 (1979). Nucleic acids of this invention, including those disclosed herein, could be generated using a conventional DNA synthesizing apparatus, such as the Applied Biosystems Model 380A or 380B DNA synthesizers (Applied Biosystems, Inc., 850 Lincoln Center Drive, Foster City, CA 94404) using phosphoramidite chemistry, thereafter ligating the fragments so as to reconstitute the entire gene. Alternatively, phosphotriester chemistry may be employed to synthesize the nucleic acids of this invention. *(See, e.g.,* M.J. Gait, ed., Oligonucleotide Synthesis, A Practical Approach, (1984)).

In an alternative methodology, namely PCR, the DNA sequences disclosed and described herein, can be produced from a plurality of starting materials. For example, starting with a cDNA preparation (e.g. cDNA library) derived from a tissue that expresses an Scarface 2 gene, suitable oligonucleotide primers complementary to, for example, SEQ ID NO:1, or to a sub-region therein, are prepared as described in U.S. Patent No. 4,889,818, hereby incorporated by reference. Other suitable protocols for the PCR are disclosed in PCR Protocols: A Guide to Method and Applications, Ed. Michael A. Innis et al., Academic Press, Inc. (1990). Using PCR, any region of the Scarface 2 gene(s) can be targeted for amplification such that full or partial length gene sequences may be produced.

The ribonucleic acids of the present invention may be prepared using polynucleotide synthetic methods discussed *supra,* or they may be prepared enzymatically, for example, using RNA polymerase to transcribe a Scarface 2 DNA template.

The most preferred systems for preparing the ribonucleic acids of the present invention employ the RNA polymerase from the bacteriophage T7 or the bacteriophage SP6. These RNA polymerases are highly specific, requiring the insertion of bacteriophage-specific sequences at the 5' end of the template to be transcribed. *See,* Maniatis et *al., supra.*

This invention also provides nucleic acids, RNA or DNA, that are complementary to Scarface 2 genes or fragments thereof, for example, SEQ ID NO:1.

### Nucleic Acid Probes

The present invention also provides probes and primers useful, for example, in hybridization screens of genomic, subgenomic, or cDNA libraries, as well as hybridization against nucleic acids derived from cell lines or tissues. Such hybridization screens are useful as methods to identify and isolate full length, or identical, homologous or functionally related sequences from human or other organisms.

In one embodiment the present invention relates to the use of-a nucleic acid disclosed herein as a probe to identify and isolate full-length genes comprising said nucleic acids. A nucleic acid compound comprising SEQ ID NO:1, or a complementary sequence thereof, or a fragment thereof, which is at least 18 base pairs in length, and which will selectively hybridize to DNA or mRNA encoding Scarface 2 protein or fragment thereof, or a functionally related protein, is provided. Preferably, the 18 or more base pair compound is DNA. *See e.g*. B. Wallace and G. Miyada, "Oligonucleotide Probes for the Screening of Recombinant DNA Libraries," In *Meth. Enzym.,* 152, 432-442, Academic Press (1987).

Probes and primers can be prepared by enzymatic or recombinant methods, well known to those skilled in the art (*See e.g.* Sambrook et al. *supra*)*.* A probe may be a single stranded nucleic acid sequence which is complementary in some particular degree to a nucleic acid sequence sought to be detected ("target sequence"). A probe may be labeled with a detectable moiety such as a radio-isotope, antigen, or chemiluminescent moiety. A description of the use of nucleic acid hybridization as a procedure for the detection of particular nucleic acid sequences is described in U.S. Patent No. 4,851,330 to Kohne, entitled "Method for Detection, Identification and Quantitation of Non-Viral Organisms."

Having the DNA sequence of the present invention allows preparation of relatively short DNA (or RNA) sequences having the ability to specifically hybridize to nucleic acids that are homologous or identical to sequences disclosed herein. In these aspects, nucleic acid probes of an appropriate length are prepared. The ability of such nucleic acid probes to specifically hybridize to a polynucleotide encoding a Scarface 2 gene or related sequence lends particular utility in a variety of embodiments. Most importantly, the probes may be used in a variety of assays for detecting the presence of complementary sequences in a given sample.

In certain embodiments, it is advantageous to use oligonucleotide primers. The sequence of such primers is designed using a polynucleotide of the present invention for use in detecting, amplifying or mutating a defined segment of a gene or polynucleotide that encodes an Scarface 2 polypeptide, using PCR technology.

Preferred nucleic acid sequences employed for hybridization studies, or assays, include probe molecules that are complementary to at least an about 18 to an about 70-nucleotide long stretch of a polynucleotide that encodes an Scarface 2 polypeptide. Molecules having complementary sequences over stretches greater than 18 bases in length are generally preferred, in order to increase stability and selectivity of the hybrid. One will generally prefer to design nucleic acid molecules having gene-complementary stretches of 25 to 40 nucleotides, 55 to 70 nucleotides, or even longer where desired. Such fragments may be readily prepared by, for example, directly synthesizing the fragment by chemical means, by application of nucleic acid reproduction technology, such as the PCR TM technology of U.S. Pat. No. 4,603,102, herein incorporated by reference, or by excising selected DNA fragments from recombinant plasmids containing appropriate inserts and suitable restriction enzyme sites.

The following guidelines are useful for designing probes with desirable characteristics. The extent and specificity of hybridization reactions are affected by a number of factors that determine the sensitivity and specificity of a particular probe, whether perfectly complementary to its target or not. The affect of various experimental parameters and conditions are well known to those skilled in the art.

First, the stability of the probe:target nucleic acid hybrid should be chosen to be compatible with the assay conditions. This may be accomplished by avoiding long A and T rich sequences, by terminating the hybrids with G:C base pairs and by designing a probe with an appropriate Tm (i.e. melting temperature). The melting profile, including the Tm of a hybrid comprising an oligonucleotide and target sequence, may be determined using a Hybridization Protection Assay. The probe should be chosen so that the length and % GC content result in a Tm about 2°-10° C higher than the temperature at which the final assay will be performed. The base composition of the probe is also a significant factor because G-C base pairs exhibit greater thermal stability as compared to A-T base pairs. Thus, hybridization involving complementary nucleic acids of higher G-C content will be more stable at higher temperatures.

The ionic strength and incubation temperature under which a probe will be used should also be taken into account. It is known that hybridization will increase as the ionic strength of the reaction mixture increases, and that the thermal stability of molecular hybrids will increase with increasing ionic strength. On the other hand, chemical reagents such as formamide, urea, DMSO and alcohols, which disrupt hydrogen bonds, increase the stringency of hybridization. Destabilization of hydrogen bonds by such reagents can greatly reduce the Tm. In general, optimal hybridization for synthetic oligonucleotide probes of about 10-50 bases in length occurs approximately 5° C below the melting temperature for a given duplex. Incubation at temperatures below the optimum may allow mismatched base sequences to hybridize and can therefore result in reduced specificity.

The length of the target nucleic acid sequence and, accordingly, the length of the probe sequence can also be important. In some cases, there may be several sequences from a particular region, varying in location and length, which will yield probes with the desired hybridization characteristics. In other cases, one sequence may be significantly better than another even though the one sequence differs merely by a single base. Finally, there can be intramolecular and intermolecular hybrids formed within a probe if there is sufficient self-complementarity. Such structures can be avoided through careful probe design. Computer programs are available to search for this type of interaction.

A probe molecule may be used for hybridizing to a sample suspected of possessing a Scarface 2 or Scarface 2 related nucleotide sequence. The hybridization reaction is carried out under suitable conditions of stringency.

Alternatively, such DNA molecules may be used in a number of techniques including their use as: (1) diagnostic tools to detect polymorphisms in DNA samples from a human or other mammal; (2) means for detecting and isolating homologs of Scarface 2 and related polypeptides from a DNA library potentially containing such sequences; (3) primers for hybridizing to related sequences for the purpose of amplifying those sequences; and (4) primers for altering the native Scarface 2 DNA sequences; as well as other techniques which rely on the similarity of the DNA sequences to those of the Scarface 2 DNA segments herein disclosed.

Once synthesized, oligonucleotide probes may be labeled by any of several well known methods. *See e.g.* Maniatis *et.al.,* Molecular Cloning (2d ed. 1989). Useful labels include radioisotopes, as well as non-radioactive reporting groups. Isotopic labels include H³, S³⁵, P³², I¹²⁵, Cobalt, and C¹⁸. Most methods of isotopic labeling involve the use of enzymes and include methods such as nick-translation, end-labeling, second strand synthesis, and reverse transcription. When using radio-labeled probes, hybridization can be detected by autoradiography, scintillation counting, or gamma counting. The detection method selected will depend upon the hybridization conditions and the particular radio isotope used for labeling.

Non-isotopic materials can also be used for labeling, and may be introduced internally into the sequence or at the end of the sequence. Modified nucleotides may be incorporated enzymatically or chemically, and chemical modifications of the probe may be performed during or after synthesis of the probe, for example, by the use of non-nucleotide linker groups. Non-isotopic labels include fluorescent molecules, chemiluminescent molecules, enzymes, cofactors, enzyme substrates, haptens or other ligands. In a preferred embodiment of the invention, the length of an oligonucleotide probe is greater than or equal to about 18 nucleotides and less than or equal to about 50 nucleotides. Labeling of an oligonucleotide of the present invention may be performed enzymatically using [³²P]-labeled ATP and the enzyme T4 polynucleotide kinase.

### Vectors

Another aspect of the present invention relates to recombinant DNA cloning vectors and expression vectors comprising the nucleic acids of the present invention. The preferred nucleic acid vectors are those which comprise DNA, for example, SEQ ID NO:1.

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene desired in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids well known to the skilled artisan.

When preparing an expression vector the skilled artisan understands that there are many variables to be considered, for example, whether to use a constitutive or inducible promoter. The practitioner also understands that the amount of nucleic acid or protein to be produced dictates, in part, the selection of the expression system. Regarding promoter sequences, inducible promoters are preferred because they enable high level, regulatable expression of an operably-linked gene. The skilled artisan will recognize a number of suitable promoters that respond to a variety of inducers, for example, carbon source, metal ions, and heat. Other relevant considerations regarding an expression vector include whether to include sequences for directing the localization of a recombinant protein. For example, a sequence encoding a signal peptide preceding the coding region of a gene is useful for directing the extracellular export of a resulting polypeptide.

The present invention also provides a method for constructing a recombinant host cell capable of expressing the proteins of the invention, said method comprising transforming or otherwise introducing into a host cell a recombinant DNA vector that comprises an isolated DNA sequence that encodes a protein of this invention. A suitable host cell is any eucaryotic cell that can accomodate high level expression of an exogenously introduced gene or protein, and that will incorporate said protein into its membrane structure. Vectors for expression are those which comprise an Scarface 2 gene or fragment thereof, e.g. SEQ ID NO:1. Transformed host cells may be cultured under conditions well known to skilled artisans such that a protein of the present invention is expressed, thereby producing a recombinant Scarface 2 protein in the recombinant host cell.

For the purpose of identifying compounds having utility as modifiers of angiogenesis, it would be desirable to identify compounds that bind an Scarface 2 protein of the present invention, and/or modify its activity. A method for determining agents that bind the Scarface 2 protein and/or inhibit its activity, comprises contacting the Scarface 2 protein with a test compound and monitoring binding by any suitable means.

The instant invention provides a screening system for discovering compounds that bind the Scarface 2 protein, said screening system comprising the steps of:
a) preparing Scarface 2 protein;
b) exposing said Scarface 2 protein to a test compound;
c) quantifying the binding of said compound to Scarface 2 protein by any suitable means, for example, by monitoring a change in tyrosine kinase activity.

Utilization of the screening system described above provides a means to identify compounds that may alter the biological function of Scarface 2 proteins. This screening method may be adapted to large-scale, automated procedures such as a PANDEX® (Baxter-Dade Diagnostics) system, allowing for efficient high-volume screening for potential therapeutic agents.

In one embodiment, Scarface-2 is prepared as described herein, preferably using recombinant DNA technology. A test compound is introduced into a reaction vessel containing an Scarface-2 protein or fragment thereof. Binding of Scarface-2 by a test compound is determined by any suitable means. For example, in one method radioactively- labeled or chemically-labeled test compound may be used. Binding of the protein by the test compound is assessed, for example, by quantifying bound label versus unbound label using any suitable method.

Binding of a test compound may also be carried out by a method disclosed in U.S. Patent 5,585,277, which hereby is incorporated by reference. In this method, binding of a test compound to a protein is assessed by monitoring the ratio of folded protein to unfolded protein, for example, by monitoring sensitivity of said protein to a protease, or amenability for binding of said protein by a specific antibody against the folded state of the protein.

The foregoing screening methods are useful for identifying an antagonist of a Scarface-2 protein as a lead to a pharmaceutical compound for treating cancer. For example, a compound that binds Scarface-2, or related fragment thereof, is identified by combining a test compound with Scarface-2 under conditions that cause the protein to exist in a ratio of folded to unfolded states. If a test compound binds the folded state of the protein, the relative amount of folded protein will be higher than in the case of a test compound that does not bind the protein. The ratio of protein in the folded versus unfolded state is easily determinable by, for example, susceptibility to digestion by a protease, or binding to a specific antibody, or binding to chaperonin protein, or binding to any suitable surface.

### Therapeutic Applications

In one embodiment, the present invention relates to therapeutic applications for Scarface 2 and analogs thereof. For example, Scarface 2 can be administered alone or in combination with other Scarface proteins to enhance wound healing such as in enhancing post-surgical recovery. In another embodiment, Scarface 2 can be used to inhibit tumor growth and metastasis, and to prevent the onset of tumor growth or tumorigenesis comprising the administration of an therapeutically effective amount of Scarface 2.

For therapeutic use, an effective amount of Scarface 2 protein is administered to an organism in need thereof in a dose between about 1 and 1000 ug/kg body weight. In practicing the methods contemplated, Scarface 2 or analog can be administered in a single daily dose or in multiple doses per day. The amount per administration will be determined by the physician and depend on such factors as the nature and severity of the disease, and the age and general health of the patient.

In another embodiment, the present invention relates to methods for treating soft tissue cancers such as leukemia comprising the administration of an therapeutically effective amount of Scarface 2 to a patient in need thereof.

In another embodiment, the present invention relates to therapeutic applications in which Scarface 2 or analog thereof is administered as a treatment for cardiac blockage.

In another embodiment, the invention relates to amplification of stem cells. Amplification of stem cells is useful for a variety of clinically relevant procedures, including, for example, transfusion procedures, or promoting the effectiveness of bone marrow transplants. The invention therefore further relates to a method for amplifying stem cells by treatment with Scarface 2, e.g. hematopoietic stem cells. In this aspect, the invention comprises administering Scarface 2 peptide or analog thereof to amplify stem cells, in vivo or in vitro. For example, hematopoietic stem cell amplification may be achieved in vitro by exposure of bone marrow derived cells to Scarface-2 peptide.

The present invention also provides pharmaceutical compositions comprising as the active agent a Scarface-2 polypeptide, fragment, analog thereof, or a pharmaceutically acceptable non-toxic salt thereof, and a pharmaceutically acceptable solid or liquid carrier. A pharmaceutical composition can also comprise as the active agent a Scarface-2 antagonist or agonist, and a pharmaceutically acceptable solid or liquid carrier. For example, compounds comprising Scarface-2 can be admixed with conventional pharmaceutical carriers and excipients, and used in the form of tablets, capsules, elixirs, suspensions, syrups, wafers, and the like. The compositions comprising Scarface-2 will contain from about 0.1% to 90% by weight of the active compound, and more generally from about 10% to 30%. The compositions may contain common carriers and excipients such as corn starch or gelatin, lactose, sucrose, microcrystalline cellulose, kaolin, mannitol, dicalcium phosphate, sodium chloride, and alginic acid. The compounds can be formulated for oral or parenteral administration.

For intravenous (IV) use, the Scarface 2 protein is administered in commonly used intravenous fluid(s) and administered by infusion. Such fluids, for example, physiological saline, Ringer's solution or 5% dextrose solution can be used.

For intramuscular preparations, a sterile formulation, preferably a suitable soluble salt form of the Scarface 2 protein, for example SEQ ID NO:2, such as the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate.

Skilled artisans will recognize that IC₅₀ values are dependent on the selectivity of the compound tested. For example, a compound with an IC₅₀ which is less than 10 nM is generally considered an excellent candidate for drug therapy. However, a compound which has a lower affinity, but is selective for a particular target, may be an even better candidate. The skilled artisan will recognize that any information regarding the binding potential, inhibitory activity, or selectivity of a particular compound is useful toward the development of pharmaceutical products.

The following examples more fully describe the present invention. Those skilled in the art will recognize that the particular reagents, equipment, and procedures described are merely illustrative and are not intended to limit the present invention in any manner.

### EXAMPLE 1

### RT-PCR Amplification of Scarface-2 from mRNA

The Scarface-2 gene is isolated by reverse transcriptase PCR (RT-PCR) using conventional methods. Total RNA from a tissue that expresses the Scarface-2 gene, for example, muscle tissue, is prepared using standard methods. First strand cDNA synthesis is achieved using a commercially available kit (SuperScript™ System; Life Technologies) by PCR in conjunction with specific primers directed at any suitable region of SEQ ID NO:1.

Amplification is carried out by adding to the first strand cDNA (dried under vacuum): 8 µl of 10X synthesis buffer (200 mM Tris-HCl, pH 8.4; 500 mM KCl, 25 mM MgCl₂, 1 ug/ul BSA); 68 µl distilled water; 1 µl each of a 10 uM solution of each primer; and 1 µl Taq DNA polymerase (2 to 5 U/µl). The reaction is heated at 94° C for 5 min. to denature the RNA/cDNA hybrid. Then, 15 to 30 cycles of PCR amplification are performed using any suitable thermal cycle apparatus. The amplified sample may be analyzed by agarose gel electrophoresis to check for an appropriately-sized fragment.

### EXAMPLE 2

### Production of a Vector for Expressing Scarface-2 in a Host Cell

An expression vector suitable for expressing Scarface-2 or fragment thereof in a variety of procaryotic host cells, such as *E*. *coli* is easily made. The vector contains an origin of replication (Ori), an ampicillin resistance gene (Amp) useful for selecting cells which have incorporated the vector following a tranformation procedure, and further comprises the T7 promoter and T7 terminator sequences in operable linkage to a Scarface-2 coding region. Plasmid pET11A (obtained from Novogen, Madison WI) is a suitable parent plasmid. pET11A is linearized by restriction with endonucleases NdeI and BamHI. Linearized pET11A is ligated to a DNA fragment bearing NdeI and BamHI sticky ends and comprising the coding region of the Scarface-2 gene as disclosed by SEQ ID NO:1.

The Scarface-2 gene used in this construction may be slightly modified at the 5' end (amino terminus of encoded protein) in order to simplify purification of the encoded protein product. For this purpose, an oligonucleotide encoding 8 histidine residues is inserted at or near the 5' end of SEQ ID NO:1, so as to maintain the correct reading frame. Placement of the histidine residues at the amino terminus of the encoded protein serves to enable the IMAC one-step protein purification procedure.

### EXAMPLE 3

### Recombinant Expression and Purification of Scarface-2 Protein

An expression vector that carries a gene encoding Scarface-2 or fragment thereof and which gene is operably-linked to an expression promoter is transformed into *E. coli* BL21 (DE3) *(hsdS gal cIts857 ind*1*Sam*7*nin*5*lac*UV5-T7gene 1) using standard methods. Transformants, selected for resistance to ampicillin, are chosen at random and tested for the presence of the vector by agarose gel electrophoresis using quick plasmid preparations. Colonies which contain the vector are grown in L broth and the protein product encoded by the vector-borne gene is purified by immobilized metal ion affinity chromatography (IMAC), essentially as described in US Patent 4,569,794.

Briefly, the IMAC column is prepared as follows. A metal-free chelating resin (e.g. Sepharose 6B IDA, Pharmacia) is washed in distilled water to remove preservative substances and infused with a suitable metal ion [e.g. Ni(II), Co(II), or Cu(II)] by adding a 50mM metal chloride or metal sulfate aqueous solution until about 75% of the interstitial spaces of the resin are saturated with colored metal ion. The column is then ready to receive a crude cellular extract containing the recombinant protein product.

After removing unbound proteins and other materials by washing the column with any suitable buffer, pH 7.5, the bound protein is eluted in any suitable buffer at pH 4.3, or preferably with an imidizole-containing buffer at pH 7.5.

### EXAMPLE 4

### Tissue Distributuion of Scarface-2 mRNA

Scarface-2 mRNA is detected in a mammalian tissue by Northern analysis. Total RNA from different tissues is isolated by a standard guanidine chloride/phenol extraction method, and poly-A⁺ RNA isolated using oligo(dT)-cellulose type 7 (Pharmacia). Electrophoresis of RNA samples is carried out in formaldehyde followed by capillary transfer to Zeta-Probe™ nylon membranes (Bio-Rad, Hercules, Calif.). SEQ ID NO:1 is used as a template for generating probes using a MultiPrime™ random priming kit (Amersham, Arlington Heights, Ill.). The efficiency of the labeling reaction is approximately 4 x 10¹⁰ cpm incorporated per µg of template. The hybridization buffer contained 0.5M sodium phosphate, 7% SDS (wt/vol), 1% BSA (wt/vol), and 1 mM EDTA. Prehybridization is carried out in hybridization buffer at 65° C for 2 h, and ³²P-labeled probe is added and incubation continued overnight. Filters were washed in Buffer A (40 mM sodium phosphate pH 7.2, 5% SDS [wt/vol], 0.5% BSA [wt/vol], and 1 mM EDTA) at 65° C for 1 h, then in Buffer B (40 mM sodium phosphate, pH 7.2, 1% SDS [wt/vol], and 1 mM EDTA) at 65° C for 20 min. Washed filters were air-dried and exposed to Kodak X-OMAT AR film at -80° C with an intensifying screen.

### EXAMPLE 5

### Detecting Ligands that Bind Scarface-2 Using a Chaperonin Protein Assay

The wells of an ELISA plate are coated with chaperonin by incubation for several hours with a 4 ug/ml solution of the protein in Tris-buffered Saline (TBS: 10 mM Tris-HCl, pH7.5, 0.2M NaCl). Plates are washed 3 times with TBS containing 0.1% Tween-20 (TBST). Then, a mixture of Scarface-2 protein (sufficient amount to saturate about 50% of the binding sites on chaperonin) and test compound (10⁻⁹ to 10⁻⁵ M) in about 50 µl volume is added to each well of the plate for an incubation of about 60 minutes. Aliquots from each well are then transferred to the wells of fresh plates and incubation is continued for 60 minutes at room temperature, followed by 3 washes with TBST. Next, about 50 µl of an antibody specific for Scarface-2 in 5% nonfat dry milk is added to each well for 30 minutes at room temperature. After washing, about 50 µl of goat anti-rabbit IgG alkaline phosphatase conjugate at an appropriate dilution in TBST plus 5% nonfat dry milk is added to each will and incubated 30 minutes at room temperature. The plates are washed again with TBST, and 0.1 ml of 1 mg/ml p-nitrophenylphosphate in 0.1% diethanolamine is added. Color development (proportional to bound alkaline phosphatase antibody conjugate) is monitored with an ELISA plate reader. When binding by the test ligand has occurred, ELISA analysis reveals Scarface-2 in solution at higher concentrations than in the absence of test ligand.

### EXAMPLE 6

### Production of an Antibody to Scarface-2 Protein

Substantially pure Scarface-2 protein or fragment thereof is isolated from transfected or transformed cells using any of the well known methods in the art, or by a method specifically disclosed herein. Concentration of protein is adjusted, for example, by filtration through an Amicon filter device such that the level is about 1 to 5 ug/ml. Monoclonal or polyclonal antibody is prepared as follows.

Monoclonal antibody can be prepared from murine hybridomas according to the method of Kohler and Milstein (*Nature,* 256, 495, 1975), or a modified method thereof. Briefly, a mouse is repetitively inoculated with a few micrograms of the Scarface-2 protein (e.g. SEQ ID NO:2) or fragment thereof, or fusion peptide thereof, over a period of a few weeks. The mouse is then sacrificed, and antibody producing cells of the spleen are isolated. Spleen cells are fused with mouse myeloma cells using polyethylene glycol. Fused cells that produce antibody are identified by any suitable immunoassay, for example, ELISA, as described in E. Engvall, *Meth. Enzymol.,* 70, 419, 1980.

Polyclonal antiserum can be prepared by well known methods *(See e.g.* J. Vaitukaitis *et.al. Clin. Endocirnol. Metab.* 33, 988, 1971) that involve immunizing suitable animals with a Scarface-2 protein, fragments thereof, or fusion proteins thereof. Small doses (e.g. nanogram amounts) of antigen administered at multiple intradermal sites are the most reliable method.

### EXAMPLE 7

### Assay For Scarface-2 Activity

Scarface-2 activity is assayed by measuring tyrosine-specific phosphorylation, essentially as described in S. Davis *et al., Cell,* 87, 1161-1169, 1996; and T. Stitt *et. al. Cell,* 80, 661-670, 1995, both of which are herein incorporated by reference. Briefly, assays are done using late-confluency T-75 flasks of human umbilical vein endothelial cells (available from Clonetics, Inc. San Diego, CA). Cells are serum starved in high glucose DMDM for 2 - 4 hr. Conditioned medium, plus and minus varying amounts of Scarface-2 protein from about 10 ng/ml to about 10 ug/ml, is placed on cells for 5 - 10 min. at 37°C. Cells are then lysed in 1% NP-40 in PBS containing 1 mM PMSF, 0.14 U/ml aprotinin, 1 mM EDTA, and 1 mM sodium orthovanadate. The lysates are immunoprecipitated with a phosphotyrosine specific rabbit antiserum, electrophoresed, and immunoblotted (Western Blot) to nylon membranes. Following incubation of the blots with HRP-conjugated goat anti-rabbit antibodies ( Caltag, Inc.) bands are visualized by ECL.

### EXAMPLE 8

### Assay For Scarface-2 Antagonist

Assay reactions are set up essentially as described in Example 7, accept that a compound to be tested for Scarface-2 antagonist activity is included at the step of adding Scarface-2 to conditioned medium for 5 - 10 min. prior to cell lysis. Multiple assays can be set up in which a constant amount of Scarface-2 is incubated with varying amounts of test compound, for example from about 10 ng/ml to about 100 ug/ml.

### EXAMPLE 9

### Identification of Critical Regions and Functional Analogs of Scarface 2

Critical regions of the Scarface 2 molecule can be identified by any number of suitable in vitro mutagenesis techniques, well known to the skilled artisan. One suitable method, known as Alanine-scanning mutagenesis (See Cunningham and Wells, Science, 244, 1081-1085, 1989), allows the introduction of individual alanine residues at each position of Scarface 2, as a test for the criticality of the replaced wild-type residue. The resulting mutated Scarface 2 molecules can be tested for retention of biological activity, for example, by assessing their ability to phosphorylate Tie 2.

The skilled artisan also recognizes that specific mutations can be constructed at defined sites in the wild-type Scarface 2 molecule to produce species or analogs of Scarface 2 falling within the genus defined by SEQ ID NO:3. The resulting analogs can be tested without undue experimentation for function, for example, by the ability of the Scarface 2 analog(s) to stimulate the phosphorylation of Tie 2. In this aspect, mutations defined according to SEQ ID NO:3 are targeted to any appropriate region of a wild-type Scarface 2. For example, using the cDNA defined by SEQ ID NO:1, PCR oligonucleotide mutagenesis techniques, well known to the skilled artisan, can be applied to introduce mutations in SEQ ID NO:1 such that the following single-substitution Scarface 2 analogs are constructed by the above method(s), wherein, the residue at position 278 is changed from Lys to Ala; the residue at position 281 is changed from Gln to Ala; the residue at position 295 is changed from Met to Ala; the residue at position 307 is changed from Leu to Ala; the residue at position 313 is changed from Leu to Ala; the residue at position 337 is changed from Asn to Ala; the residue at position 340 is changed from Lys to Ala; the residue at position 358 is changed from Met to Ala; the residue at position 368 is changed from Leu to Ala; the residue at position 378 is changed from Lys to Ala; the residue at position 379 is changed from Val to Ala, and the residue at position 384 is changed from Ser to Ala. The resulting Scarface 2 analogs are tested for biological activity, for example, the ability to phosphorylate Tie 2 or to stimulate the phosphorylation of Tie 2, or any other suitable assay, for example the stimulation of calcium influx in endothelial cells.

### EXAMPLE 10

### Endothelial Cell Calcium Influx Assay

Human venous umbilical vein endothelial cells (USVEC, available from Cell Systems) are grown in appropriate media (50:50 without glycine, 1% glutamine, 10 mM Hepes, 10% FBS, 10 ng/ml bFGF). Cells are plated on 96-well plates at a density of about 2 x 10⁴ per well and washed three times with HBSS plus 10 mM Hepes buffer. Samples of Scarface 2 or analog thereof is added to the cells and the plates run on an FLIPR machine. The fluorescence change is measured and expressed relative to a positive control, for example, ionomycin.

## Claims

1. A substantially pure protein having an amino acid sequence that is at least 70% identical to SEQ ID NO: 2, or fragment thereof, said protein exhibiting Scarface 2-like activity in vivo or in vitro.

2. A substantially pure protein having an amino acid sequence that is encoded by a nucleic acid sequence that hybridizes to SEQ ID NO:1 under high stringency conditions said protein having Scarface 2 activity.

3. An analog of Scarface 2 having a sequence that is SEQ ID NO:3.

4. A substantially pure Scarface 2 protein having an amino acid sequence identified herein as SEQ ID NO: 2.

5. An isolated nucleic acid compound encoding a protein of Claim 1, Claim 2, Claim 3, or claim 4.

6. An isolated nucleic acid compound that encodes a Scarface 2 protein wherein said nucleic acid is SEQ ID NO:1.

7. A vector comprising an isolated nucleic acid claim 5 or 6.

8. A host cell containing a vector of Claim 7.

9. A pharmaceutical formulation comprising as an active ingredient an Scarface 2 protein or peptide, associated with one or more pharmaceutically acceptable carriers, excipients, or diluents thereof.

10. Scarface 2, as claimed in any one of claims 1 to 5, or a pharmaceutically acceptable salt thereof, for use as a pharmaceutical.
